# EUROPÄISCHE PATENTSCHRIFT

(11) **EP 3 441 977 B1**
(45) Veröffentlichungstag und Bekanntmachung des Hinweises auf die Patenterteilung: **07.08.2024**
(21) Anmeldenummer: 17185322.9
(22) Anmeldetag: 08.08.2017
(51) Int. Cl.: G16H 50/50, A61B 34/20, G16H 30/40, G16H 40/63, A61B 90/00, A61B 17/00, A61B 34/10

(54) **VERFAHREN UND SYSTEM ZUR UNTERSTÜTZUNG VON MEDIZINISCHEM PERSONAL**
METHOD AND SYSTEM FOR SUPPORTING MEDICAL PERSONNEL
PROCÉDÉ ET SYSTÈME D'AIDE AU PERSONNEL MÉDICAL

(43) Veröffentlichungstag der Anmeldung: 13.02.2019
(73) Patentinhaber: Siemens Healthineers AG, 91301 Forchheim (DE)
(72) Erfinder: Blendinger, Heinz, 91315 Höchstadt/Aisch (DE)
(74) Vertreter: Siemens Healthineers Patent Attorneys

(56) Entgegenhaltungen:
- DE-A1- 102004 030 836
- DE-A1- 102012 224 057
- US-A1- 2008 137 923
- US-A1- 2016 191 887
- JUNE-GOO LEE ET AL: "Deep Learning in Medical Imaging: General Overview", KOREAN JOURNAL OF RADIOLOGY, 19 May 2017 (2017-05-19), pages 570 - 584, XP055444120, Retrieved from the Internet <URL:https://www.ncbi.nlm.nih.gov/pmc/articles/PMC5447633/pdf/kjr-18-570.pdf> [retrieved on 20180124], DOI: 10.3348/kjr.2017.18.4.570

## Beschreibung

Die Erfindung betrifft ein Verfahren und ein System zur Unterstützung von medizinischem Personal bei einer Prozedur an einem Patienten.

Man ist heutzutage bemüht, sowohl diagnostische als auch interventionelle Prozeduren mit möglichst geringer Verletzung des jeweiligen Patienten durchzuführen. Während einer interventionellen Prozedur im OP-Umfeld, beispielsweise während eines minimal-invasiven Eingriffs mittels eines Katheters, ist üblicherweise ein jeweils aktiver, also die Prozedur durchführender, Chirurg oder Arzt durch eine bildliche Darstellung auf einem Monitor über einen jeweils aktuellen Status des Eingriffs informiert. Hierfür kann beispielsweise eine kontinuierliche bildliche Erfassung eines Eingriffsbereiches mittels eines Röntgengeräts vorgesehen sein. Es kann dabei nachteilig sein, dass der Chirurg seinen Blick aktiv von der realen Situation, also dem Katheter, seinen Händen und dem Patienten, abwenden und auf den Monitor richten muss, um die reale, äußerlich sichtbare Situation und seine Bewegungen bei der Führung des Katheters mit der internen, auf dem Monitor dargestellten Situation - also beispielsweise der Position der Katheterspitze - abzugleichen. Weiteres anwesendes medizinisches Personal kann sich oftmals nur unzureichend oder ungenau durch Beobachtung der bildlichen Darstellung über den aktuellen Status und Verlauf der Prozedur informieren. Personen, die mit der Art der bildlichen Darstellung nicht vertraut sind und/oder den Monitor nicht einsehen können, müssen oftmals in fehleranfälliger Art und Weise durch andere Personen informiert werden. Es kann beispielsweise eine zusätzliche und nachteilige Belastung des jeweils aktiven Arztes bedeuten, zusätzlich zu seiner für das Patientenwohl kritischen Arbeit der Platzierung des Katheters das anwesende medizinische Personal zu informieren oder anzuweisen. Eine Anpassung der Position des Monitors, sodass dieser für mehr Personal einfacher einsehbar ist, kann zu einer suboptimalen Sichtbarkeit oder Ablesbarkeit des Monitors für den aktiven Arzt führen, was ebenfalls als nachteilig einzustufen ist.

Aus der DE 10 2012 224 057 A1 sind bereits ein Verfahren und eine Vorrichtung zur Bildunterstützung bekannt. Das dortige Verfahren umfasst eine präoperative Erzeugung eines 3D-Bilddatensatzes eines Navigationsbereiches durch ein Bildgebungsverfahren und daraus Erzeugen eines 3D-Modell einer Patientenanatomie. Weiter wird intraoperativ ein 2D-Durchleuchtungsbild des Navigationsbereiches erzeugt. Darin wird eine Bildposition zumindest eines Teils eines medizinischen Instruments bestimmt, beispielsweise manuell oder mittels geeigneter Bildverarbeitungsmethoden. Ebenfalls intraoperativ wird das 2D-Durchleuchtungsbild mit dem 3D-Bilddatensatz registriert. In Abhängigkeit von der bestimmten Bildposition und Eigenschaften des medizinischen Instruments wird dann zumindest ein Teil von diesem in das 3D-Modell eingeblendet. Die Einblendung kann dabei erfolgen in Abhängigkeit vorbekannter Gewebeeigenschaften eines Gewebes, in welches das medizinische Instrument eingebracht wird. In Abhängigkeit einer bestimmten zukünftigen Bewegung und/oder Deformation können ideale Verbringungsparameter für das Instrument bestimmt und auf einer Anzeigeeinrichtung oder akustisch wiedergegeben werden. Dabei kann ein behandelnder Arzt wie bei einem Navigationssystem angeleitet werden.

Die DE 10 2004 030 836 A1 beschreibt ein Verfahren zur Bilddarstellung eines in einen sich rhythmisch bewegenden Untersuchungsbereich eines Patienten eingebrachten medizinischen Instruments. Dabei wird ein präoperativer 3D-Bilddatensatz des Untersuchungsbereiches mit zugeordneten EKG-Daten zur Erzeugung eines 3D-Rekonstruktionsbildes des sich bewegenden Untersuchungsbereiches verwendet. Es wird dann lediglich ein aktuelles 2D-Durchleuchtungsbild des Untersuchungsbereichs aufgenommen und es werden dazu EKG-Daten erfasst. Anhand der EKG-Daten erfolgt eine Registrierung des 3D-Bilddatensatzes mit dem 2D-Durchleuchtungsbild. Danach werden kontinuierlich aktuelle EKG-Daten aufgenommen und über diese die Darstellung des 3D-Rekonstruktionsbildes getriggert. Zudem werden kontinuierlich in einem Koordinatensystem, das mit dem Koordinatensystem des 3D-Rekonstruktionsbildes registriert ist, die aktuellen Positionsdaten des Instruments mittels eines Navigationssystems aufgenommen und das Instrument lagerichtig in dem 3D-Rekonstruktionsbild dargestellt. Um Änderungen der Position des Untersuchungsbereiches zu erfassen können intermittierend weitere 2D-Durchleuchtungsbilder und die zugehörigen EKG-Daten aufgenommen werden, anhand welcher die Registrierung und die lagerichtige Darstellung überprüft und gegebenenfalls korrigiert werden.

Aus der Publikation June-Goo Lee ET AL: "Deep Learning in Medical lmaging: General Overview", Korean Journal of Radiology, 19. Mai 2017 (2017-05-19), Seiten 570-584, XP055444120, DOI: 10.3348/kjr.2017.18.4.570 (gefunden im Internet: URL:https://www.ncbi.nlm.nih.gov/pmc/articles/PMC5447633/pdf/ kjr-18-570.pdf) ist es bekannt, mittels künstlicher Intelligenz medizinische Bilddaten automatisch zu analysieren und klinische relevante Bildinhalte zu identifizieren.

Aus US 2016/0191887 A1 ist die Verwendung stereoskopischer Bilddaten bekannt, um eine Augmented Reality Darstellung für die Chirurgie zu erzeugen. Für die Darstellung werden sowohl pre-operative als auch intra-operative Bilddaten sowie Augmented Reality Videodaten gemeinsam dargestellt.

Aus US 2008/0137923 A1 ist ein Verfahren bekannt, um medizinische Instrumente in Bilddaten hervorzuheben. Zu diesem Zweck werden der Darstellung Modell-Darstellungen des medizinischen Instruments überlagert.

Aufgabe der vorliegenden Erfindung ist es, durch Unterstützung von medizinischem Personal einen verbesserten Ablauf von medizinischen Prozeduren zu ermöglichen.

Diese Aufgabe wird erfindungsgemäß durch die Gegenstände der unabhängigen Patentansprüche gelöst. Vorteilhafte Ausgestaltungen und Weiterbildungen der Erfindung sind in den abhängigen Patentansprüchen sowie in der Beschreibung und in den Zeichnungen angegeben.

Ein erfindungsgemäßes Verfahren zur Unterstützung von medizinischem Personal bei einer Prozedur oder bei einem Eingriff an einem Patienten umfasst mehrere Verfahrensschritte. Zunächst und während der Prozedur werden mittels eines medizinischen bildgebenden Verfahrens erzeugte Bilddaten des Patienten und eines medizinischen Objekts kontinuierlich erfasst. Aus den beziehungsweise unter Verwendung der Bilddaten wird ein digitales Patientenmodell, also ein Modell des Patienten, erzeugt und kontinuierlich aktualisiert. Eine jeweils aktuelle Position des medizinischen Objekts wird kontinuierlich nachverfolgt. Dies kann anhand der Bilddaten erfolgen. Ebenfalls als Teil des Verfahrens werden ein situativer und räumlicher Kontext, in dem sich das medizinische Objekt befindet, durch automatisches Verarbeiten der Bilddaten mittels eines Bildverarbeitungsalgorithmus bestimmt. Dabei kann der situative Kontext durch die Art der jeweils aktuell durchgeführten Prozedur, die Art des medizinischen Objekts oder einen Zustand des Patienten gegeben sein. Basierend auf dem bestimmten Kontext werden aus dem Patientenmodell, also anhand des oder ausgehend von dem oder basierend auf dem Patientenmodell, automatisch Sprachdaten erzeugt. Diese Sprachdaten beschreiben wenigstens einen Teil des bestimmten Kontextes, also der jeweiligen Situation, in Sprachform. Die automatisch erzeugten Sprachdaten werden dann an das medizinische Personal ausgegeben. Dieses Ausgeben erfolgt mittels einer akustischen Sprachausgabe und/oder als Text auf einer Anzeigefläche. Als das medizinische Objekt wird ein Katheter verwendet. Es werden unterschiedliche Sprachdaten erzeugt und in Abhängigkeit von ihrem Inhalt in unterschiedliche Kategorien betreffend ein Anatomiemerkmal oder das medizinische Objekt eingeordnet. Es werden nur Sprachdaten aus ausgewählten der Kategorien ausgegeben.

Das Verfahren kann bei vielfältigen medizinischen Prozeduren und Eingriffen angewendet werden. Dementsprechend kann auch das medizinische Objekt auf vielfältige unterschiedliche Arten und Weisen ausgeprägt oder ausgebildet sein. Ohne die vorliegende Erfindung hierauf zu beschränken, kann das Verfahren besonders vorteilhaft beispielsweise bei einem diagnostischen oder interventionellen minimal-invasiven Einsatz eines Katheters angewendet werden. Das medizinische Objekt kann also beispielsweise der Katheter oder ein Teil davon sein. Ebenso kann das medizinische Objekt jedoch ein chirurgisches oder allgemein ein medizinisches Instrument, ein Implantat, eine Gefäßstütze (Stent), ein Werkzeug, eine Schraube, ein Draht, ein Schlauch und/oder dergleichen sein. Letztlich sollte das medizinische Objekt mittels des bildgebenden Verfahrens erfassbar sein, also sichtbar gemacht werden können.

Im Sinne der vorliegenden Erfindung kann sich das kontinuierliche Erfassen der Bilddaten sowohl auf ein Aufnehmen oder Erzeugen der Bilddaten, also ein Abbilden des Patienten, beziehen als auch auf ein Abrufen oder Auslesen der Bilddaten, beispielsweise von einem bilderzeugenden Gerät oder einem Datenspeicher. Dass die Bilddaten kontinuierlich erfasst werden, muss im Sinne der vorliegenden Erfindung dabei keinen Dauerstrichbetrieb implizieren, sondern kann ebenso ein regelmäßiges, wiederholtes Erfassen der beziehungsweise von Bilddaten bedeuten. Beispielsweise kann automatisch eine Serie von Röntgenbildern mit einer bestimmten Frequenz aufgenommen werden. Die Bilddaten werden also nicht nur zu einem einzigen Zeitpunkt, sondern zu mehreren Zeitpunkten in einem zeitlichen Verlauf erfasst. Die Bilddaten können also insbesondere einen zeitlichen Verlauf oder eine zeitliche Entwicklung abbilden.

Die Bilddaten können jeweils einen Teilbereich des Patienten und/oder des medizinischen Objekts abbilden oder darstellen. Zum Erzeugen der Bilddaten können dabei unterschiedliche bildgebende Verfahren eingesetzt werden. So können die Bilddaten beispielsweise Röntgen-, Ultraschall-, Angiographie- oder Tomographiebilder sein. Ebenso können die Bilddaten zwei- oder dreidimensional sein beziehungsweise eine Serie von 2D- oder 3D-Bildern umfassen.

Das Patientenmodell kann ein anhand der Bilddaten oder anhand von Rohdaten rekonstruiertes Bild zumindest eines Teilbereiches des Patienten und des ebenfalls abgebildeten medizinischen Objekts sein. Da sowohl zumindest ein Teilbereich des Patienten als auch des medizinischen Objekts in den Bilddaten enthalten beziehungsweise abgebildet sind, beinhaltet auch das Patientenmodell eine Abbildung oder Repräsentation zumindest eines Teils des Patienten und des medizinischen Objekts. Somit kann das Patientenmodell also eine räumliche Lagebeziehung zwischen zumindest einem Teil des Patienten und dem medizinischen Objekt darstellen oder beschreiben. Das Patientenmodell kann ein dreidimensionales Modell sein. Ebenso kann das Patientenmodell dynamisch sein, also eine Bewegung, insbesondere eine Relativbewegung, des Patienten, eines Teils des Patienten und/oder des medizinischen Objekts modellieren, also abbilden und/oder beschreiben. So kann das Patientenmodell beispielsweise eine Herz- oder Atembewegung des Patienten und/oder einen Vorschub des Katheters in dem Patienten modellieren, also berücksichtigen und/oder veranschaulichen.

Das Patientenmodell kann also eine ganz oder teilweise bildliche Darstellung sein. Dabei kann das Patientenmodell eine unterstützende Visualisierung oder Veranschaulichung enthalten. Dies kann beispielsweise eine automatisch oder manuell erzeugte Markierung, Hervorhebung, Einfärbung, Beschriftung oder dergleichen sein. Es kann beispielsweise möglich sein, die Bewegung des medizinischen Objekts beziehungsweise dessen Pfad den Bilddaten überlagert darzustellen, um eine bessere Erkennbarkeit oder Auswertbarkeit zu erreichen.

Ebenso kann das Patientenmodell im Sinne der vorliegenden Erfindung eine mathematische und/oder grafische Beschreibung oder Modellierung zumindest eines Teilbereiches des Patienten und des medizinischen Objekts sein oder umfassen. Es ist beispielsweise möglich, das medizinische Objekt zu identifizieren, also dessen Art oder Modell zu bestimmen. Dann kann aus einer entsprechenden Modelldatenbank beispielsweise ein CAD-Modell des medizinischen Objekts abgerufen und in dem beziehungsweise für das Patientenmodell ver- oder eingearbeitet werden. Dann kann beispielsweise ein den Patienten beschreibender Teil des Patientenmodells aus den Bilddaten erzeugt und diesem Teil dann das virtuelle Modell des medizinischen Objekts überlagert werden, wobei die jeweils aktuelle Position des medizinischen Objekts ebenfalls aus den Bilddaten bestimmt werden kann. Das Patientenmodell kann also basierend auf verschiedenen Datenquellen aufgebaut werden. Hierdurch kann das Patientenmodell besonders genau sein und es kann vorteilhaft eine besonders einfache automatische Weiterverarbeitung und Auswertung des Patientenmodells ermöglicht werden.

Allgemein kann durch die Verwendung des Patientenmodells vorteilhaft eine Bündelung sämtlicher verfügbarer relevanter Daten und Informationen eben in dem Patientenmodell erreicht werden. Beim Aktualisieren des Modells können die jeweils aktuellen Bilddaten, also beispielsweise das jeweils zuletzt aufgenommene Röntgenbild, in das Patientenmodell eingerechnet werden. Dabei kann beispielsweise ein bisher noch nicht abgebildeter und lediglich schematisch modellierter Teil des Patienten in dem Patientenmodell durch das dann verfügbare reale Röntgenbild ersetzt werden. Ebenso kann beispielsweise eine Bildqualität des Patientenmodells durch Berücksichtigung der jeweils aktuellen Bilddaten verbessert werden, indem diese etwa zur Verbesserung einer Rauschunterdrückung verwendet, insbesondere mit den bis zum jeweils aktuellen Zeitpunkt bereits kumulativ erfassten Bilddaten kombiniert, werden. Das Patientenmodell ermöglicht vorteilhaft auch eine besonders flexible Auswertung sämtlicher erfasster Daten. So ist beispielsweise die Visualisierung und/oder die Auswertung nicht auf eine bisher oftmals übliche flüchtige Live-Darstellung beschränkt, da die erfassten Bilddaten sowie gegebenenfalls weitere Daten oder Informationen in das Patientenmodell einfließen und somit nicht - abgesehen von einer Live-Wiedergabe - ungenutzt verworfen werden.

Das Nachverfolgen (Tracking) der Position des medizinischen Objekts kann beispielsweise durch einen Positionsgeber an dem medizinischen Objekt selbst und/oder durch eine Bildauswertung beziehungsweise Bildverarbeitung der Bilddaten erfolgen. Dies kann vorteilhaft erleichtert werden durch Markierungen oder Marker, die an dem medizinischen Objekt angeordnet sein können. Die Art dieser Markierungen oder Marker kann dabei abhängig sein von dem verwendeten bildgebenden Verfahren, sodass jeweils sichergestellt ist, dass die Markierungen oder Marker mittels des jeweiligen bildgebenden Verfahrens erfasst beziehungsweise abgebildet, also dargestellt werden können. Ebenso können manuelle An- oder Eingaben zum Nachverfolgen der Position des medizinischen Objekts erfasst und verarbeitet oder ausgewertet werden. So kann beispielsweise das medizinische Personal manuell an- oder eingeben, dass das medizinische Objekt eine bestimmte Position oder Stellung erreicht hat. Hierdurch kann beispielsweise die automatische Bildauswertung zum Nachverfolgen der Position des medizinischen Objekts unterstützt beziehungsweise abgesichert werden, indem verlässliche Referenzpunkte geschaffen werden. Somit wird eine Plausibilisierung der automatischen Auswertung oder Positionsbestimmung ermöglicht.

Der Kontext, in dem sich das medizinische Objekt befindet, kann durch unterschiedliche Daten beziehungsweise Gegebenheiten charakterisiert werden beziehungsweise gegeben sein. Der räumliche Kontext kann beispielsweise angeben, an welcher Position oder Stelle, insbesondere relativ zu beziehungsweise in oder an dem Patienten, sich das medizinische Objekt befindet. So kann beispielsweise ein unterschiedlicher Kontext gegeben sein, je nachdem ob sich das medizinische Objekt, beispielsweise also der Katheter, in einem Bein oder in der Brusthöhle oder in einem bestimmten Gefäß oder an einem bestimmten Organ des Patienten befindet. Der räumliche Kontext kann ebenso durch eine Position einer Eintrittsstelle des Katheters in den Patienten und/oder eine Bewegungsrichtung relativ zu, insbesondere an oder in, dem Patienten bestimmt sein. So kann beispielsweise jeweils ein anderer Kontext gegeben sein, je nachdem ob das medizinische Objekt in dem Patienten von unten nach oben, von oben nach unten, in den Patienten hinein oder aus dem Patienten hinaus bewegt wird.

Ebenso kann der räumliche und/oder situative Kontext charakterisiert oder gegeben sein, durch eine räumliche Lage oder Körperhaltung des Patienten, durch eine Stellung oder Position, in der sich ein bildgebendes Gerät, beispielsweise das Röntgengerät, befindet beziehungsweise zu einem jeweiligen Aufnahmezeitpunkt der Bilddaten befunden hat, durch eine Umgebung des Patienten, beispielsweise je nachdem ob sich der Patient in einem Operationssaal (OP) oder in einer ambulanten oder prekären Behandlungssituation oder -umgebung befindet, oder dergleichen mehr.

Der situative Kontext kann beispielsweise durch die Art der jeweils aktuell durchgeführten Prozedur, die Art des medizinischen Objekts, einen Zustand des Patienten, also beispielsweise eine jeweilige medizinische und/oder anatomische Situation oder Besonderheit, oder dergleichen mehr gegeben oder charakterisiert sein.

Das Bestimmen beziehungsweise Berücksichtigen des Kontextes kann vorteilhaft eine situationsgerechte beziehungsweise situationsangepasste, besonders genaue, zutreffende und verlässliche automatische Auswertung und somit eine entsprechend vorteilhafte verlässliche und zutreffende Bestimmung der zu erzeugenden Sprachdaten ermöglichen.

Der Kontext kann beispielsweise automatisch bestimmt werden durch Auswertung der Bilddaten, des Patientenmodells und/oder weiterer Daten beziehungsweise Datenquellen. So kann beispielsweise eine digitale Patientenakte automatisch ausgelesen oder ausgewertet werden, um bestimmte körperliche Merkmale des Patienten und/oder die Art der Prozedur zu ermitteln.

Der Bildverarbeitungsalgorithmus kann beispielsweise eine Segmentierung, eine Kanten- oder Objekterkennung, eine Bewegungserkennung und dergleichen mehr umfassen beziehungsweise durchführen. So kann automatisch bestimmt werden, wo sich das medizinische Objekt in dem Patienten, insbesondere in welchem Abstand zu einem bestimmten Körperteil oder anatomischen Merkmal des Patienten, sich das anatomische Objekt befindet. Dies kann bereits als Kontext ausreichen. Bevorzugt kann ein detaillierterer Kontext jedoch beispielsweise zusätzlich die Information liefern, wo sich ein jeweiliger Zielpunkt oder eine jeweilige Zielposition des medizinischen Objekts in oder an dem Patienten befindet, ob die jeweilige Prozedur eine reine Bewegung des medizinischen Objekts oder eine Manipulation an dem Patienten vorsieht und/oder ob sich das medizinische Objekt entsprechend einem vorgegebenen oder idealen Verlauf oder Pfad bewegt.

Um den Kontext zu bestimmen, kann das Patientenmodell also beispielsweise zum Bestimmen der Relativposition des medizinischen Objekts bezogen auf den Patienten genutzt beziehungsweise ausgewertet werden. Dabei kann der bestimmte Kontext diese faktische Information bewertbar und/oder in einen inhaltlichen beziehungsweise anschaulichen Rahmen einordenbar machen.

Die Sprachdaten können also die jeweilige aktuelle Situation, beispielsweise die jeweils aktuelle Relativposition des medizinischen Objekts, in allgemein verständlicher anschaulicher Form beschreiben. Die Sprachdaten können dabei anschauliche, also sprachliche Begriffe oder Wörter verwenden, sodass sie sich somit vorteilhaft nicht beispielsweise in abstrakten Koordinatenangaben oder Zahlenangaben erschöpfen. Die Sprachdaten können beispielsweise in Form von Sätzen oder Satzfragmenten, also in natürlicher Sprache beziehungsweise Sprachform erzeugt werden. In konkreten Beispielen können als Sprachdaten beispielsweise die Aussagen oder Angaben erzeugt werden "36 mm vor Arteria Renalis", um beispielsweise die aktuelle Position einer Katheterspitze anzugeben, "noch 23 mm bis zur Klappenebene", um beispielsweise die aktuelle Position und Bewegungsrichtung der Katheterspitze bezogen auf einen konkreten automatisch erkannten oder vorher festgelegten Bezugspunkt - wie etwa eine Herzklappe - anzugeben, "Mitte des Stent erreicht", um eine Relation beispielsweise der Katheterspitze zu einem anderen Objekt zu beschreiben, oder "aktueller Gefäßdurchmesser 12 mm", um die Situation oder eine aktuelle Umgebung beispielsweise der Katheterspitze zu charakterisieren oder ein anatomisches Merkmal oder Detail des Patienten, insbesondere in einer Umgebung des medizinischen Objekts, zu beschreiben. Bei dem Erzeugen der Sprachdaten können außer dem bestimmten Kontext und dem Patientenmodell selbstverständlich weitere Daten, Steuersignale, Konfigurationen oder Einstellungen berücksichtigt werden.

Für die akustische Sprachausgabe der Sprachdaten können diese beispielsweise mittels eines Sprachsynthese- oder TTSverfahrens (englisch "Text-To-Speech") in Audiosignale umgewandelt werden. Diese können dann beispielsweise an eine Ausgabeeinrichtung, welche beispielsweise einen Lautsprecher aufweisen kann, übermittelt werden. Die akustische Sprachausgabe ermöglicht es besonders vorteilhaft, das gesamte medizinische Personal unabhängig vom jeweiligen Standpunkt oder der Einsehbarkeit eines bestimmten Bereiches, beispielsweise eines bestimmten Bildschirms, zu informieren. Somit wird der aktive, also die Prozedur durchführende, Arzt oder Chirurg von dieser Aufgabe vorteilhaft entlastet, was zu einem Patientenwohl und einem reibungslosen, verbesserten Ablauf der Prozedur beitragen kann. Durch das zeitnahe und zuverlässige Informieren des medizinischen Personals kann dieses sich auf die jeweilige Situation einstellen, auf bevorstehende Ereignisse vorbereiten und dabei eine jeweilige, insbesondere visuelle Aufmerksamkeit auf jeweils aufgabengemäß relevante Bereiche oder Objekte richten. Hierdurch kann also der Ablauf der gesamten Prozedur und eine Organisation und Interaktion, beispielsweise im Operationssaal, verbessert werden.

Für den aktiven Arzt ist es dabei besonders vorteilhaft, dass er selbst die ausgegebenen Sprachdaten nutzen kann als Feedback beziehungsweise zur Einschätzung oder Bewertung der jeweiligen Situation. Es ist dabei ein besonderer Vorteil, dass der aktive Arzt zur intellektuellen Aufnahme der durch die akustisch ausgegebenen Sprachdaten vermittelten Informationen seinen Blick nicht von dem Patienten, einem jeweiligen von ihm bedienten Gerät oder seinen Händen abwenden muss. Hierdurch kann er die Prozedur unterbrechungsfrei, besonders schnell und sicher durchführen. Da die ausgegebenen Sprachdaten beziehungsweise die entsprechenden, darin enthaltenen Informationen durch automatische Auswertung oder Verarbeitung des Patientenmodells ermittelt werden, können diese vorteilhaft präziser oder zuverlässiger sein als eine lediglich auf Erfahrung oder Betrachtung von Röntgenbildern oder dergleichen basierende Einschätzung des jeweiligen aktiven Arztes oder sonstigen medizinischen Personals. Während der Prozedur steht üblicherweise weder Zeit noch Personal zur Verfügung, um beispielsweise manuell in den Röntgenbildern beispielsweise einen jeweiligen Abstand zu vermessen, sodass die vorliegende Erfindung auch Daten bereitstellen kann, die bisher oftmals nicht während der Prozedur verfügbar waren.

Die Ausgabe der Sprachdaten als Text auf einer Anzeigefläche kann beispielsweise zur Unterstützung der akustischen Sprachausgabe beziehungsweise als Referenz zur Absicherung dienen, beispielsweise für den Fall, dass eine Person die akustische Sprachausgabe nicht zuverlässig verstanden hat. Aber auch die Ausgabe der Sprachdaten ausschließlich als Text kann vorteilhaft sein. So kann durch die rein textbasierte Ausgabe eine akustische Ablenkung vermieden und gleichzeitig die Ausgabe der präzisen, automatisch ermittelten, modellbasierten Daten ermöglicht werden.

Technisch kann die Ausgabe der Sprachdaten sowohl in akustischer als auch in visueller Form auf unterschiedliche Arten und Weisen realisiert werden. Beispielsweise ist eine Ausgabe über Raumlautsprecher ebenso möglich wie eine Ausgabe über jeweilige individuelle Kopfhörer des medizinischen Personals. Dabei kann beispielsweise auch eine Individualisierung der jeweils ausgegebenen Sprachdaten vorgesehen sein. Das bedeutet, dass - beispielsweise in Abhängigkeit von einer jeweiligen Aufgabe der jeweiligen Person - die jeweilige Person nur eine auf sie abgestimmte, also beispielsweise für ihre jeweilige Aufgabe relevante Auswahl, also Untermenge, aller erzeugten beziehungsweise verfügbaren Sprachdaten erhält, also ausgegeben bekommt. Dies wird durch das automatische Erzeugen der Sprachdaten ermöglicht beziehungsweise vereinfacht, da eine automatische Kategorisierung und Zuordnung besonders schnell vorgenommen werden kann. Die Ausgabe der Sprachdaten als Text kann beispielsweise in Form eines Logbuches, beispielsweise auf einem separaten Bildschirm oder in einem eigenen Anzeigebereich der Anzeigefläche erfolgen. Dadurch kann vorteilhaft jederzeit eine Historie, also ein Verlauf der Prozedur nachvollzogen werden. Ebenso ist es beispielsweise möglich den Text als Untertitel zu den jeweiligen Bilddaten einzublenden. Ebenso ist es möglich, den Text in einem Laufband als durchlaufenden Text - vergleichbar einem Newsticker - auszugeben. Hierdurch kann vorteilhaft eine Größe eines zur Darstellung benötigten Anzeigebereiches minimiert und dabei sichergestellt werden, dass stets die aktuellsten Sprachdaten angezeigt werden.

In vorteilhafter Ausgestaltung der vorliegenden Erfindung wird als der Kontext oder als Teil des Kontextes ein abgebildeter Teilbereich des Patienten und/oder ein anatomisches Merkmal des Patienten und/oder eine räumlich-geometrische Relation zwischen dem medizinischen Objekt und wenigstens einem Umgebungsdetail in einer Umgebung des medizinischen Objekts bestimmt. Die räumlich-geometrische Relation kann insbesondere ein Abstand des medizinischen Objekts zu einem vorbestimmten und/oder automatisch erkannten anatomischen Merkmal des Patienten sein. Mit anderen Worten kann also, insbesondere automatisch, bestimmt werden, welcher Teilbereich des Patienten in den Bilddaten abgebildet ist - beispielsweise ein Bein, ein unterer oder oberer Torsobereich, ein Arm oder der Kopf des Patienten. Dies kann durch Auswertung der Bilddaten und/oder durch Berücksichtigung oder Auswertung weiterer Datenquellen erreicht werden. So kann etwa ein Zugriff auf eine Datenbank ebenso erfolgen wie ein Erfassen oder Auslesen einer, beispielsweise durch das medizinische Personal manuell vorgenommenen, Benutzereingabe und/oder eine Auswertung von Geometriedaten. Die Geometriedaten können beispielsweise eine Lage des Patienten und/oder des die Bilddaten generierenden Geräts, beispielsweise also eine bestimmte Anordnung und Stellung des Patienten in einem C-Bogen-Röntgengerät, beschreiben oder angeben. Die Bestimmung des abgebildeten Teilbereiches des Patienten ermöglicht vorteilhaft eine Einschränkung und Spezifizierung der Möglichkeiten, um welche anatomischen Details es sich bei bestimmten Features der Bilddaten handeln kann. Aufgrund der jeweiligen Geometrie kann beispielsweise ausgeschlossen werden, dass bestimmte Teile des Patienten in den jeweiligen Bilddaten abgebildet sind, so dass eine zuverlässigere Identifizierung ermöglicht wird. Hierdurch kann also die automatische Objekterkennung verbessert werden.

Das anatomische Merkmal des Patienten kann beispielsweise ein bestimmtes Anatomiedetail in der Umgebung des medizinischen Objekts sein, wodurch die genaue Verortung oder Lokalisierung des medizinischen Objekts vereinfacht wird. Durch das Bestimmen des anatomischen Merkmals kann dieses vorteilhaft in den Sprachdaten erwähnt werden, um dem medizinischen Personal eine verbesserte und vereinfachte Einordnung der ausgegebenen Sprachdaten beziehungsweise der jeweils aktuellen Situation zu ermöglichen, da hierdurch die Situation besonders anschaulich beschrieben werden kann. Ebenso kann das anatomische Merkmal eine bestimmte individuelle Ausbildung eines Körperteils oder eines Anatomiedetails oder beispielsweise eine bestimmte Verletzung des Patienten sein. Hierdurch kann vorteilhaft beispielsweise ein möglicher Bewegungsspielraum für das medizinische Objekt oder eine Abweichung zu einem Normalzustand ermittelt werden. Dies kann dann vorteilhaft beispielsweise bei der automatischen Identifizierung einzelner Features in den Bilddaten und/oder bei dem Erzeugen der Sprachdaten berücksichtigt werden. Ebenso kann basierend hierauf beispielsweise ein bestimmter Sollverlauf der Prozedur als Referenz aus einer Datenbank ausgewählt und/oder ein bereitgestellter Sollverlauf der Prozedur unter Berücksichtigung oder in Abhängigkeit von dem anatomischen Merkmal modifiziert werden. Dies kann vorteilhaft eine Präzisierung der Sprachdaten beziehungsweise der durch diese übermittelten Informationen ebenso ermöglichen, wie beispielsweise eine automatische Warnung an das medizinische Personal.

Durch das Bestimmen der räumlich-geometrischen Relation zwischen dem medizinischen Objekt und dem wenigstens einen Umgebungsdetail - welches beispielsweise das oder ein anderes anatomisches Merkmal sein kann - kann das medizinische Personal, insbesondere bei einer Bewegung des medizinischen Objekts, besonders vorteilhaft über die oftmals von außen nicht oder nur schwierig erkennbare Relativposition des medizinischen Objekts informiert werden, ohne dass die Aufmerksamkeit von dem medizinischen Objekt selbst und/oder von dem Patienten abgewendet werden müsste. Dies kann besonders vorteilhaft sein, um ein unbeabsichtigtes Verletzen des Patienten durch das medizinische Objekt zu vermeiden.

In weiterer vorteilhafter Ausgestaltung der vorliegenden Erfindung wird der Kontext oder ein Teil des Kontextes bestimmt durch einen automatischen Abgleich der Bilddaten und/oder des Patientenmodells mit einer bereitgestellten Anatomiedatenbank. Mit anderen Worten kann also zur Bestimmung des Kontextes ein Vergleich der Bilddaten und/oder des Patientenmodells oder eines jeweiligen Teils davon mit beispielsweise in der Anatomiedatenbank oder einem anatomischen Katalog oder Atlas gespeicherten Beispielbildern oder Modelldaten durchgeführt werden. Dabei kann beispielsweise eine einfache Differenzbildung, ein Schwellenwertverfahren, eine Mustererkennung oder eine Ähnlichkeitsanalyse beziehungsweise Ähnlichkeitsbewertung durchgeführt werden. Dies kann beispielsweise mittels eines herkömmlichen Bildverarbeitungsalgorithmus oder etwa mittels eines neuronalen Netzes realisiert werden. Vorteilhaft kann so also der Kontext ganz oder teilweise automatisch bestimmt werden, wodurch ein Aufwand an manuellen Bedienhandlungen ebenso minimiert werden kann wie eine Fehleranfälligkeit.

In weiterer vorteilhafter Ausgestaltung der vorliegenden Erfindung werden zum Erzeugen des Patientenmodells und/oder zum Bestimmen des Kontextes eine Lage des Patienten und/oder dessen Umgebung, insbesondere eine relative Position eines Patientenlagers und/oder eines die Bilddaten generierenden Geräts, beschreibende Geometriedaten verwendet. Dies kann vorteilhaft eine zuverlässigere automatische Bestimmung oder Identifizierung der durchgeführten Prozedur ebenso ermöglichen, wie eine automatische Bestimmung oder Vorhersage einer erwarteten Lage oder eines erwarteten Verlaufs eines oder mehrerer anatomischer Merkmale des Patienten. Beispielsweise kann sich ein Verlauf und/oder ein Durchmesser eines Gefäßes oder eine Form eines Organs in Abhängigkeit von der Lage des Patienten ändern, je nachdem ob der Patient sich in Bauch-, Rücken- oder Seitenlage befindet. Ebenso kann die Berücksichtigung der Geometriedaten vorteilhaft zur genaueren und zuverlässigeren Identifizierung des abgebildeten Teilbereiches des Patienten beziehungsweise von in den Bilddaten abgebildeten oder dargestellten anatomischen Details beziehungsweise Merkmalen verwendet werden. Beispielsweise kann durch Auswertung der relativen Lage oder Position des die Bilddaten generierenden Geräts, also beispielsweise des Röntgengeräts, und/oder der Lage des Patienten und/oder des Patientenlagers eine Anzahl von Möglichkeiten dafür, welche anatomischen Merkmale durch die Bilddaten abgebildet sein können reduziert oder eingeschränkt werden. Ebenso kann beispielsweise basierend auf den Geometriedaten automatisch ein eingeschränkter Teilbereich der Anatomiedatenbank vorgegeben werden, der für den automatischen Abgleich der Bilddaten zur Bestimmung des Kontextes berücksichtigt wird. Insgesamt kann durch die Berücksichtigung beziehungsweise Verwendung der Geometriedaten eine genauere und zuverlässigere und/oder detailliertere Bestimmung des Kontextes ermöglicht sowie ein genaueres Patientenmodell erzeugt werden.

Die Geometriedaten können beispielsweise durch Erfassen und Auslesen einer Benutzereingabe, durch Abruf eines jeweiligen aktuellen Zustandes des die Bilddaten generierenden Geräts und/oder durch Verarbeitung und Auswertung von Sensordaten gewonnen werden. Die Sensordaten können beispielsweise von Lage-, Position-, Beschleunigung- oder Bewegungssensoren, von bilderfassenden Sensoren, von einem Laserscanner und/oder dergleichen mehr erfasst beziehungsweise bereitgestellt werden.

In einer vorteilhaften Weiterbildung der Erfindung werden der Patient und/oder eine Umgebung des Patienten mittels einer 3D-Kamera räumlich erfasst. Entsprechende von der 3D-Kamera bereitgestellte Kameradaten werden dann zum Erzeugen des Patientenmodells und/oder zum Bestimmen des Kontextes verarbeitet. Mit anderen Worten kann also beispielsweise die Lage oder Haltung des Patienten mittels der 3D Kamera räumlich erfasst werden. Dies ermöglicht es vorteilhaft, das Patientenmodell besonders schnell und wirklichkeitsgetreu zu erzeugen, was wiederum eine besonders präzise Beschreibung der jeweiligen Situation durch die Sprachdaten ermöglicht. Durch das Erfassen der Umgebung des Patienten und eine entsprechende Auswertung der entsprechenden Kameradaten kann vorteilhaft beispielsweise automatisch bestimmt werden, in welchem Raum oder in welcher Art von Raum sich der Patient befindet, welche Geräte bei der Prozedur zu welchem Zeitpunkt eingesetzt werden und/oder welche Personen des medizinischen Personals anwesend und/oder an der Prozedur beteiligt sind. So kann es beispielsweise möglich sein durch eine Bildverarbeitung der Kameradaten automatisch eine Relativposition beziehungsweise eine relative Lage des Patienten zu dem die Bilddaten generierenden Geräts, also beispielsweise zu dem Röntgengerät, zu bestimmen. Dies kann wiederum vorteilhaft beispielsweise ermöglichen, automatisch den abgebildeten Teilbereich des Patienten zu identifizieren. Dazu können die Kameradaten beispielsweise in die genannten Geometriedaten umgerechnet werden.

In weiterer vorteilhafter Ausgestaltung der vorliegenden Erfindung wird das medizinische Objekt mittels eines automatischen Objekterkennungsalgorithmus und/oder durch einen automatischen Abgleich der Bilddaten mit einer bereitgestellten Objektdatenbank identifiziert. Dem identifizierten Objekt zugeordnete Objektdaten werden dann aus der Objektdatenbank oder einer anderen Datenbank abgerufen und zum Erzeugen des Patientenmodells und/oder zum Bestimmen des Kontextes verarbeitet. Mit anderen Worten kann also automatisch ermittelt werden, nicht nur wo sich das medizinische Objekt befindet, sondern um was für ein medizinisches Objekt oder um welches medizinische Objekt es sich handelt. Beispielsweise kann das medizinische Objekt hinsichtlich seiner Art oder hinsichtlich seines konkreten Modells identifiziert werden. Durch die Identifizierung des medizinischen Objekts kann beispielsweise eine präzisere Modellierung des medizinischen Objekts und/oder dessen Interaktion mit dem Patienten ermöglicht werden.

Ebenso kann die Identifikation des medizinischen Objekts verwendet werden, um beispielsweise die Art der Prozedur und/oder ein Sollverhalten des medizinischen Objekts zu bestimmen. Wird beispielsweise das medizinische Objekt als Katheter identifiziert, so entspricht es einem erwarteten Verhalten oder Sollverhalten, dass sich der Katheter in einem Gefäß des Patienten bewegt. Dies kann vorteilhaft genutzt werden, um die Sprachdaten besonders präzise und besonders zuverlässig zu erzeugen, sodass diese die jeweilige Situation besonders genau und zutreffend beschreiben. Dazu können auch die Objektdaten beitragen, welche beispielsweise eine genaue Form und Größe des medizinischen Objekts und eine genaue Position von Markierungen oder Markern an dem medizinischen Objekt, beispielsweise relativ zu einem Endpunkt angeben können. Kann beispielsweise lediglich die Position des oder der Marker oder Markierungen des medizinischen Objekts automatisch anhand der Bilddaten ermittelt werden, so kann daraus unter Berücksichtigung der abgerufenen Objektdaten präzise beispielsweise eine aktuelle Position einer Spitze oder eines Endpunkte des medizinischen Objekts berechnet werden, da die Objektdaten die Lage der Marker ebenso umfassen können wie die genaue Größe und Form des medizinischen Objekts. Diese präzise Angabe kann dann in Form der Sprachdaten ausgegeben werden.

In weiterer vorteilhafter Ausgestaltung der vorliegenden Erfindung wird als das medizinische Objekt ein Katheter verwendet. Während der Katheter durch ein Gefäß des Patienten geführt wird, wird dabei mittels eines Bildverarbeitungsalgorithmus automatisch ein Durchmesser des Gefäßes an einer jeweils aktuellen Position des Katheters, insbesondere einer Spitze des Katheters, bestimmt. Der jeweils bestimmte Durchmesser kann dann als Teil der Sprachdaten ausgegeben werden. Ebenso kann der bestimmte Durchmesser als Eingangsgröße oder Berechnungsgrundlage zur Bestimmung einer oder mehrerer anderer Größen, zum Charakterisieren der Position des medizinischen Objekts oder dergleichen verwendet werden. Diese können dann beim Erzeugen der Sprachdaten berücksichtigt, beispielsweise also durch die Sprachdaten beschrieben oder angegeben werden. Es kann beispielsweise automatisch berechnet und angegeben werden, dass die Katheterspitze in einen Abschnitt des Gefäßes gelangt ist, dessen Durchmesser kleiner oder größer als der Katheter beziehungsweise dessen Durchmesser ist. Der Durchmesser des Gefäßes kann beispielsweise kontinuierlich oder in regelmäßigen vorgegebenen zeitlichen und/oder räumlichen Abständen bestimmt werden.

In den Sprachdaten kann der bestimmte Gefäßdurchmesser aus- oder angegeben werden beispielsweise in vorgegebenen zeitlichen Abständen, jeweils nachdem der Katheter eine vorgegebene Strecke oder Entfernung in dem Gefäß zurückgelegt hat und/oder wenn sich der Durchmesser des Gefäßes um einen vorgegebenen absoluten oder relativen Betrag - beispielsweise gegenüber einem vorgegebenen Wert, einem bestimmten Messwert oder einem Durchschnittswert - verändert hat und/oder wenn der Durchmesser des Gefäßes einen vorgegebenen Schwellenwert unter- oder überschreitet. Hierdurch kann vorteilhaft eine Anzahl an durch die Sprachdaten an- beziehungsweise ausgegebenen Detail- oder Einzeldaten reduziert werden. Gleichzeitig kann sichergestellt werden, dass das medizinische Personal an jeweils relevanten Stellen über den Durchmesser des Gefäßes informiert ist. Durch das automatische Bestimmen des Durchmessers des Gefäßes, also des Gefäßdurchmesser, wird das medizinische Personal davon entlastet, den Gefäßdurchmesser selbst manuell zu ermitteln.

Vorteilhaft können die bestimmten Werte des Gefäßdurchmessers gespeichert werden. Hieraus kann dann vorteilhaft ein Profil des jeweiligen Gefäßes erstellt werden. Das Profil kann eine nützliche Referenz darstellen. Ebenso kann es möglich sein, dieses Profil als Basis für eine automatische Diagnose oder Bewertung eines Zustandes des Patienten zu verwenden. Durch das automatische Bestimmen des Gefäßdurchmesser können dabei praktikabel und mit vernachlässigbarem Zeit- und Rechenaufwand eine Vielzahl von Messwerten des Gefäßdurchmesser bestimmt werden, wodurch ein signifikant genaueres Profil erstellt werden kann als mittels einer manuellen Vermessung.

Um den Gefäßdurchmesser automatisch zu bestimmen, kann der Katheter als Referenz verwendet werden. Beispielsweise kann eine Linie oder Strecke erzeugt beziehungsweise berechnet werden, die senkrecht zu einer Haupterstreckungsrichtung des Katheters durch die Katheterspitze verläuft und eine Wand des Gefäßes an zwei einander gegenüberliegenden Punkten trifft oder schneidet. Der Gefäßdurchmesser kann dann einfach bestimmt werden als Länge der Strecke oder Linie zwischen deren Schnittpunkten mit der Gefäßwand. Der Gefäßdurchmesser kann dem medizinischen Personal beispielsweise als wichtige Größe dienen, um die Position des Katheters beurteilen oder einschätzen zu können. Beispielsweise kann durch Verfolgen der Angaben des Gefäßdurchmessers präzise ein Anfangs-, Mittel- und/oder Endpunkt einer Stenose bestimmt und der Katheter entsprechend präzise platziert werden. Ebenso kann anhand des Gefäßdurchmesser beispielsweise eine Gefäßgabelung erkannt und/oder das medizinische Personal darauf hingewiesen werden, dass besondere Vorsicht geboten ist, wenn beispielsweise der Gefäßdurchmesser besonders klein wird, beispielsweise unterhalb einen vorgegebenen Schwellenwert fällt, da in einem solchen Fall eine Bewegung des Katheters besonders leicht zu einer Verletzung des Gefäßes führen kann. Gerade in solchen Fällen kann das automatische Bestimmen des Gefäßdurchmesser ebenso wie die automatische Sprachausgabe des entsprechenden Wertes besonders vorteilhaft sein, insbesondere auch dann, wenn beispielsweise aufgrund einer jeweiligen Bildqualität der Bilddaten der Gefäßdurchmesser für das medizinische Personal selbst nicht durch einen kurzen Blick eindeutig und zuverlässig bestimmbar oder erkennbar ist. Eine längere Unterbrechung der Bewegung des Katheters, um beispielsweise die Bilddaten ausgiebig zu studieren, kann dann eine Verletzungsgefahr erhöhen, was durch die vorliegende Erfindung vermieden werden kann.

In weiterer vorteilhafter Ausgestaltung der vorliegenden Erfindung wird bei dem kontinuierlichen Erfassen der Bilddaten zwischen zwei, insbesondere zu Dokumentationszwecken, aufgenommenen Dokumentationsbildern des Patienten eine Vielzahl von Durchleuchtungsbildern mit reduzierter Strahlungsintensität aufgenommen. Diese Durchleuchtungsbilder werden dann zum Aktualisieren des Patientenmodells verarbeitet. Mit anderen Worten werden die Dokumentationsbildern also mit einer ersten Strahlungsintensität aufgenommen oder erzeugt und die Durchleuchtungsbilder mit einer zweiten Strahlungsintensität, wobei die zweite Strahlungsintensität geringer ist als die erste Strahlungsintensität. Durch dieses Vorgehen können also aufgrund der höheren Strahlungsintensität die Dokumentationsbilder möglichst genau und gut erkennbar relevante Details, Schlüsselpositionen oder Schlüsselzeitpunkte während der Prozedur abbilden beziehungsweise erfassen. Gleichzeitig kann der Patient kontinuierlich während der Prozedur abgebildet beziehungsweise bildlich erfasst werden, wobei eine Belastung des Patienten durch die reduzierte Strahlungsintensität für die Durchleuchtungsbilder minimiert wird. Die Dokumentationsbilder und die Durchleuchtungsbilder sind dabei Teil der Bilddaten.

Die reduzierte Strahlungsintensität kann für jedes einzelne der Durchleuchtungsbilder zu einer verringerten Bildqualität führen. Dies kann vorteilhaft jedoch dadurch ausgeglichen werden, dass das Patientenmodell jeweils kumulativ auf allen bis zum jeweiligen aktuellen Zeitpunkt erfassten Bilddaten, also den Dokumentationsbildern und den Durchleuchtungsbildern, basiert. Hier können also durch die besonders große und stetig wachsende Datenbasis für das Patientenmodell trotz der reduzierten Strahlungsintensität für die Durchleuchtungsbilder beispielsweise gegenüber jedem einzelnen der Durchleuchtungsbilder ein reduziertes Rauschen beziehungsweise ein verbessertes Signal-Rauschverhältnis und somit eine verbesserte Bildqualität erreicht werden. Dieses Vorgehen, die Durchleuchtungsbilder mit reduzierter Strahlungsintensität aufzunehmen, beruht zudem auf der Erkenntnis, dass während signifikanter zeitlicher Anteile der Prozedur eine maximale Bildqualität nicht unbedingt zum erfolgreichen Durchführen der Prozedur notwendig ist. Im Rahmen des erfindungsgemäßen Verfahrens kann dieses Vorgehen besonders vorteilhaft angewendet werden, da durch die automatische Bildauswertung und das automatische Nachverfolgen des medizinischen Objekts dieses trotz der reduzierten Strahlungsintensität und einer damit eventuell einhergehenden reduzierten Bildqualität besonders genau verortet beziehungsweise lokalisiert werden kann. Dies ist damit begründet, dass ein geeigneter Bildverarbeitungsalgorithmus oftmals schneller und genauer beispielsweise Objektgrenzen erkennen kann als dies einem menschlichen Beobachter zuverlässig möglich ist, insbesondere während er gleichzeitig mit anderen Aufgaben beim Durchführen der Prozedur an dem Patienten befasst ist.

Die reduzierte Strahlungsintensität ist bei dem vorliegenden Verfahren gegenüber herkömmlichen Methoden zudem deshalb weniger wichtig für das erfolgreiche Durchführen der Prozedur, weil das medizinische Personal aufgrund der Ausgabe der die jeweilige Situation beschreibenden Sprachdaten sich nicht auf ein visuelles Erkennen und seine eigene Interpretation der Bilddaten verlassen muss. Somit kann das vorliegend vorgeschlagene Vorgehen also die Strahlenbelastung für den Patienten reduzieren, ohne den Erfolg der Prozedur zu gefährden. Es ist also eine besondere Erkenntnis und ein besonderer Vorteil der vorliegenden Erfindung, die kontinuierlich während der Prozedur mit reduzierter Strahlungsintensität erfassten Durchleuchtungsbilder beziehungsweise die darin enthaltenen Daten und Informationen nicht zu verwerfen, sondern zur kontinuierlichen Aktualisierung beziehungsweise Verbesserung des Patientenmodells und somit zum Generieren einer möglichst genauen und zuverlässigen sprachlichen Beschreibung durch die Sprachdaten zu nutzen, also auszuwerten beziehungsweise zu verarbeiten.

Gemäß der vorliegenden Erfindung werden unterschiedliche Sprachdaten erzeugt und in Abhängigkeit von ihrem Inhalt in unterschiedliche Kategorien eingeordnet. Diese unterschiedlichen Kategorien können insbesondere ein Anatomiemerkmal oder das medizinische Objekt oder eine räumliche und/oder zeitliche Relation betreffen. Es werden dann nur Sprachdaten aus ausgewählten der Kategorien ausgegeben. Dass unterschiedliche Sprachdaten erzeugt werden, bedeutet dabei, dass mehrere unterschiedliche Fakten, Zustände, Bedingungen, Situationen oder dergleichen erfasst beziehungsweise ermittelt oder ausgewertet und separat voneinander durch jeweilige Sprachdaten beschrieben, also ausgedrückt oder verbalisiert werden. So kann ein Sprachdatum beispielsweise eine Positionsangabe des medizinischen Objekts sein, beispielsweise "Katheterspitze 5 mm vor markierter Herzklappenebene". Ein zweites, davon unterschiedliches Sprachdatum kann beispielsweise den Gefäßdurchmesser an der aktuellen Position der Katheterspitze angeben. Diese beiden Sprachdaten sind somit verschieden, unabhängig voneinander und betreffen unterschiedliche Fakten oder unterschiedliche Arten von Fakten. Demzufolge werden dann diese beiden Sprachdaten also in unterschiedliche Kategorien eingeordnet beziehungsweise unterschiedlichen Kategorien zugeordnet. Trifft nun beispielsweise das medizinische Personal die Auswahl, dass nur Sprachdaten der Kategorie "Anatomiemerkmal" ausgegeben werden sollen, so würde erfindungs- und verfahrensgemäß nur das zweite Sprachdatum ausgegeben werden, da dieses ein Anatomiemerkmal, nämlich den Gefäßdurchmesser beschreibt oder angibt.

Die Einordnung oder Zuordnung der Sprachdaten zu den unterschiedlichen Kategorien erfolgt also in Abhängigkeit von einer Art des jeweiligen Sprachdatums beziehungsweise in Abhängigkeit von einem Thema oder einer Bedeutung und/oder eines Bezugs beziehungsweise Bezugsobjektes des jeweiligen Sprachdatums. Um die unterschiedlichen Sprachdaten in die unterschiedlichen Kategorien einordnen zu können, können beispielsweise die jeweiligen Features oder Merkmale der Bilddaten beziehungsweise des Patientenmodells, die beim Erzeugen der jeweiligen Sprachdaten verwendet oder berücksichtigt werden beziehungsweise die den jeweiligen Sprachdaten zugrunde liegen oder auf die durch die jeweiligen Sprachdaten Bezug genommen wird, identifiziert werden. Wird beispielsweise durch den Bildverarbeitungsalgorithmus ein Abstand zwischen zwei Kanten bestimmt, so können die Objekte, zu denen die Kanten gehören beziehungsweise die durch die Kanten begrenzt werden, identifiziert werden - beispielsweise als der Katheter und eine Gefäß- oder Organwand. Es kann dann eine automatische Verschlagwortung oder ein automatischer Abgleich mit einer vorgegebenen Zuordnungstabelle vorgesehen sein, wobei die Abstandsinformation beziehungsweise das darauf basierende Sprachdatum in Abhängigkeit davon, welche Objekte identifiziert wurden beziehungsweise welche Schlagworte zugewiesen wurden, in eine oder mehrere entsprechende Kategorien eingeordnet werden.

Ebenso ist es möglich, beispielsweise unterschiedliche, spezialisierte Bildverarbeitungsalgorithmen oder Subalgorithmen beziehungsweise Subroutinen zum Ermitteln unterschiedlicher Fakten oder unterschiedlicher Arten von Fakten vorzusehen. So kann beispielsweise ein erster Algorithmus zum Bestimmen des Gefäßdurchmessers und ein zweiter Algorithmus zum Bestimmen eines Abstandes zwischen dem medizinischen Objekt und einer bestimmten Markierung und/oder einem bestimmten Anatomiemerkmal vorgesehen sein usw. Da die unterschiedlichen Algorithmen dann also stets unterschiedliche Fakten oder Situationen erfassen beziehungsweise ermitteln oder bewerten kann für jeden der Algorithmen eine automatische Einordnung von ihm ausgegebener Ausgangsdaten in eine bestimmte vorgegebene Kategorie vorgesehen sein. Hierdurch kann vorteilhaft eine besonders genaue und zuverlässige Kategorisierung ermöglicht werden.

Die Auswahl der auszugebenden Kategorien beziehungsweise Sprachdaten kann beispielsweise durch eine Benutzereingabe oder eine entsprechende Konfiguration vorgenommen werden. Ebenso kann es aber möglich sein, beispielsweise in Abhängigkeit von der Art der Prozedur, von einem jeweils aktuellen Stadium der Prozedur, von der jeweils aktuellen Position des medizinischen Objekts und/oder anderen Faktoren eine automatische Auswahl vorzunehmen. Hierdurch kann vorteilhaft sichergestellt werden, dass zu jedem Zeitpunkt beispielsweise nur für den Erfolg der Prozedur dann besonders relevante Sprachdaten ausgegeben werden, sodass das medizinische Personal nicht durch eine zu große Menge ausgegebener Sprachdaten überlastet wird. Da die Ausgabe der Sprachdaten, insbesondere bei einer akustischen Sprachausgabe, zudem einige Zeit in Anspruch nimmt, kann durch die Auswahl sichergestellt werden, dass keine Wartezeit oder Warteschlange für erzeugte aber noch nicht ausgegebene Sprachdaten entsteht. So kann beispielsweise dann wenn sich das medizinische Objekt bis auf einen vorgegebenen Abstand an ein bestimmtes Anatomiemerkmal, beispielsweise einer Herzklappe, angenähert hat, der jeweils aktuelle Abstand beziehungsweise dessen zeitnahe Ausgabe wichtiger sein als der jeweils aktuelle Gefäßdurchmesser oder eine aktuelle Geschwindigkeit des medizinischen Objekts, sodass dann automatisch nur diejenige Kategorie zur Wiedergabe der entsprechenden Sprachdaten ausgewählt wird, die eine räumliche Relation zwischen dem medizinischen Objekt und dem Patienten beziehungsweise einem bestimmten Anatomiemerkmal, hier also den Abstand, betrifft. So kann beispielsweise besonders zuverlässig verhindert werden, dass es zu einer Verletzung des Patienten durch einen unbeabsichtigten Kontakt mit dem medizinischen Objekt, beispielsweise ein Durchstoßen einer Wand oder Membran kommt.

Der beziehungsweise ein Bildverarbeitungsalgorithmus kann dazu eingerichtet sein, eine Bewegung und/oder eine Geschwindigkeit, insbesondere relativ zu dem Patienten, zu erkennen. In Abhängigkeit von der Bewegung oder der Geschwindigkeit kann dann eine automatische Auswahl der auszugebenden Sprachdaten vorgenommen werden und/oder die Ausgabe der Sprachdaten zumindest zeitweise, insbesondere für einen vorgegebenen Zeitraum, ausgesetzt werden. Hierdurch kann, insbesondere bei einer oberhalb eines vorgegebenen Schwellenwertes liegenden Geschwindigkeit, vermieden werden, dass aufgrund der zum Erzeugen und zum Ausgeben der Sprachdaten benötigten Zeit ein Zeitversatz (Lag) entsteht, wodurch die zu einem bestimmten Zeitpunkt ausgegebenen Sprachdaten nicht die jeweilige Situation an der tatsächlichen Position beschreiben würden, die das medizinische Objekt zu diesem Zeitpunkt einnimmt. So kann beispielsweise vermieden werden, dass ein bestimmter Abstand oder Gefäßdurchmesser ausgegeben wird, wenn dieser zum Zeitpunkt der entsprechenden Sprachausgabe nicht mehr aktuell ist, da sich das medizinische Objekt bereits signifikant, beispielsweise um mehr als einen vorgegebenen Schwellenwert, weiter bewegt hat gegenüber einer Position, an welcher der Abstand beziehungsweise der Gefäßdurchmesser oder dergleichen ermittelt wurde. Sinkt die Geschwindigkeit des medizinischen Objekts anschließend wieder unter den entsprechenden Schwellenwert, so kann die Ausgabe der Sprachdaten automatisch fortgesetzt beziehungsweise wieder aufgenommen werden. Zwischenzeitlich, das heißt während sich das medizinische Objekt bewegt hat, erzeugte Sprachdaten können dann beispielsweise übersprungen, also nicht ausgegeben werden.

In weiterer vorteilhafter Ausgestaltung der vorliegenden Erfindung werden die Bilddaten und/oder die Patientenmodelle einer Vielzahl von verschiedenen Patienten mittels einer Methode des maschinellen Lernens, insbesondere mittels eines neuronalen Netzes, verarbeitet und dadurch ein Normalzustand und/oder ein Normalverhalten wenigstens eines Teils des Patienten gelernt. Ein solcher Normalzustand kann beispielsweise einen Gefäßdurchmesser oder einen Bewegungsumfang einer Herzklappe oder dergleichen betreffen beziehungsweise beschreiben. Basierend auf dem gelernten Normalzustand und/oder dem gelernten Normalverhalten wird dann automatisch eine Abweichung zwischen den jeweils aktuellen Bilddaten und/oder dem jeweils aktuellen Patientenmodell einerseits und dem Normalzustand und/oder dem Normalverhalten andererseits erkannt beziehungsweise ermittelt. Weiter werden dann die Abweichung beschreibende Sprachdaten erzeugt und ausgegeben. Alternativ oder zusätzlich werden die jeweils aktuellen Bilddaten und/oder das jeweils aktuelle Patientenmodell in einer Abweichungsdatenbank gespeichert. So kann also das medizinische Personal auf erkannte Abweichungen hingewiesen werden, welche beispielsweise je nach Skalierung oder Ausschnitt von entsprechenden auf einem Bildschirm dargestellten Bilddaten schwierig erkennbar sein können. Die Abweichungsdatenbank kann also als selbstlernende Bibliothek verstanden werden. Diese kann beispielsweise als Referenz und/oder zur automatischen Diagnose oder Bewertung von Krankheitsbildern, Miss- oder Fehlbildungen oder dergleichen dienen. Die erkannte Abweichung und/oder die Abweichungsdatenbank können zum Bestimmen des Kontextes und/oder zum Erzeugen und/oder zum Aktualisieren des Patientenmodells verwendet werden. Hierdurch können die ausgegebenen Sprachdaten die tatsächliche Situation, also die Realität besonders genau und zuverlässig beschreiben.

Ein erfindungsgemäßes System zur Unterstützung von medizinischem Personal bei einer Prozedur an einem Patienten weist eine Erfassungseinrichtung, eine Datenverarbeitungseinrichtung, eine Spracherzeugungseinrichtung und eine Ausgabeeinrichtung auf. Die Erfassungseinrichtung dient beziehungsweise ist eingerichtet zum kontinuierlichen Erfassen von mittels eines medizinischen bildgebenden Verfahrens erzeugten Bilddaten des Patienten und eines medizinischen Objekts. Die Datenverarbeitungseinrichtung dient beziehungsweise ist eingerichtet zum kontinuierlichen Aktualisieren eines digitalen Patientenmodells auf Basis der jeweils aktuellen Bilddaten. Die Datenverarbeitungseinrichtung dient weiterhin beziehungsweise ist weiterhin eingerichtet zum Nachverfolgen einer Position des medizinischen Objekts und zum automatischen Bestimmen eines situativen und räumlichen Kontexts, in dem sich das medizinische Objekt befindet, durch Verarbeiten der Bilddaten mittels eines Bildverarbeitungsalgorithmus. Dabei kann der situative Kontext durch die Art der jeweils aktuell durchgeführten Prozedur, die Art des medizinischen Objekts oder einen Zustand des Patienten gegeben sein. Die Datenverarbeitungseinrichtung kann auch eingerichtet sein zum Erzeugen des digitalen Patientenmodells, beispielsweise auf Basis der Bilddaten und/oder anderer Daten oder Datenquellen. Die Spracherzeugungseinrichtung dient beziehungsweise ist eingerichtet zum Erzeugen von Sprachdaten basierend auf dem bestimmten Kontext aus dem Patientenmodell, wobei die Sprachdaten wenigstens einen Teil des bestimmten Kontextes in Sprachform beschreiben. Die Ausgabeeinrichtung dient beziehungsweise ist eingerichtet zum Ausgeben der Sprachdaten an das medizinische Personal mittels einer akustischen Sprachausgabe und/oder als Text auf einer Anzeigefläche der Ausgabeeinrichtung. Als das medizinische Objekt wird ein Katheter verwendet. Es werden unterschiedliche Sprachdaten erzeugt und in Abhängigkeit von ihrem Inhalt in unterschiedliche Kategorien betreffend ein Anatomiemerkmal oder das medizinische Objekt eingeordnet. Es werden nur Sprachdaten aus ausgewählten der Kategorien ausgegeben.

Das erfindungsgemäße System kann insbesondere eingerichtet sein zum Durchführen zumindest einer Ausführungsform des erfindungsgemäßen Verfahrens. Dazu kann das erfindungsgemäße System beispielsweise einen Datenträger aufweisen mit einem Programmcode, der die Verfahrensschritte des erfindungsgemäßen Verfahrens repräsentiert beziehungsweise kodiert. Das System kann wenigstens eine Mikrochip- oder Mikroprozessoreinrichtung aufweisen, welche diesen Programmcode ausführen kann. Das erfindungsgemäße System kann ebenso eine oder mehrere Schnittstellen und/oder wenigstens ein Benutzerinterface aufweisen, worüber Daten und/oder Eingaben empfangen beziehungsweise erfasst werden können.

Die bisher und im Folgenden angegebenen Eigenschaften und Weiterbildungen des erfindungsgemäßen Verfahrens sowie die entsprechenden Vorteile sind jeweils sinngemäß auf das erfindungsgemäße System und/oder zur Durchführung des erfindungsgemäßen Verfahrens verwendete oder verwendbare Bauteile und Einrichtungen übertragbar und umgekehrt. Es gehören also zu der Erfindung auch solche Weiterbildungen des erfindungsgemäßen Verfahrens und des erfindungsgemäßen Systems, welche Ausgestaltungen aufweisen, die hier nicht explizit in der jeweiligen Kombination beschrieben sind.

Weitere Merkmale, Einzelheiten und Vorteile der vorliegenden Erfindung ergeben sich aus der nachfolgenden Beschreibung bevorzugter Ausführungsbeispiele sowie anhand der Zeichnungen. Dabei zeigen:
- FIG 1: schematisch eine OP-Situation, in der ein Arzt durch ein System unterstützt wird; und
- FIG 2: einen beispielhaften schematischen Ablaufplan eines Verfahrens zum Unterstützen von medizinischem Personal bei einer Prozedur an einem Patienten.

Bei den im Folgenden erläuterten Ausführungsbeispielen handelt es sich um bevorzugte Ausführungsformen der Erfindung. Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren sind die beschriebenen Ausführungsformen auch durch weitere der bereits beschriebenen Merkmale der Erfindung ergänzbar.

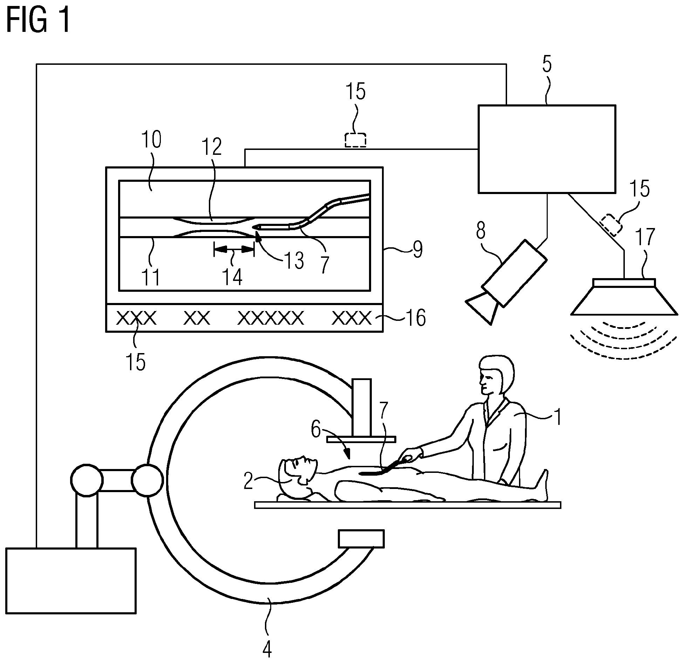

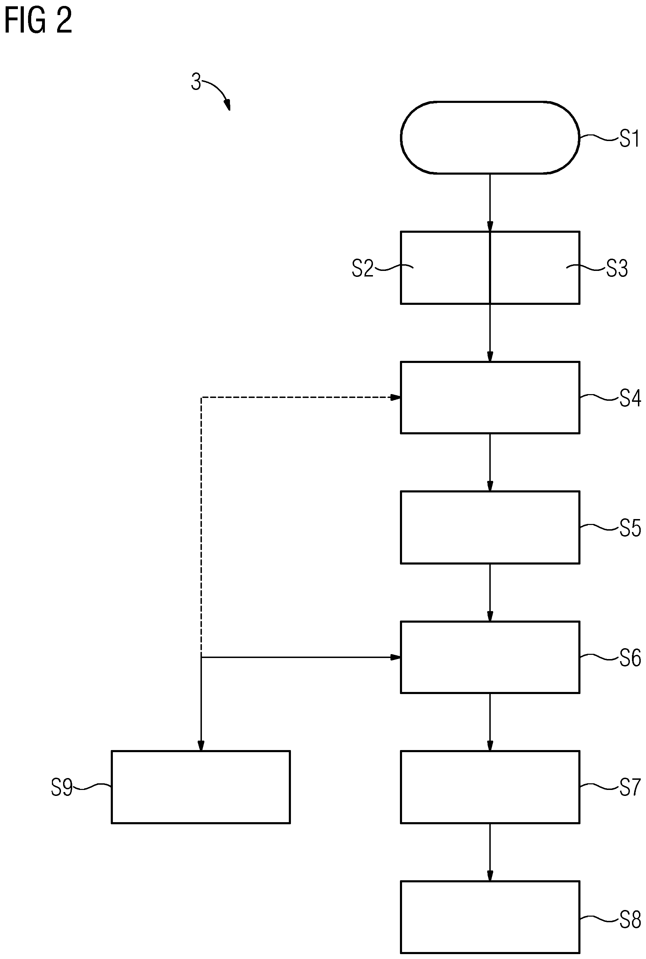

FIG 1 zeigt schematisch eine Situation in einem Operationssaal, in der medizinisches Personal, hier ein aktiver Arzt 1, durch ein System beim Durchführen einer Prozedur an einem Patienten 2 unterstützt wird. FIG 2 zeigt einen beispielhaften schematischen Ablaufplan 3 eines entsprechenden Verfahrens zur Unterstützung von medizinischem Personal, hier also des Arztes 1. Im Folgenden werden FIG 1 und FIG 2 gemeinsam erläutert.

In einem Verfahrensschritt S1 wird das Verfahren gestartet. Hier können vorbereitende Maßnahmen durchgeführt werden oder bereits durchgeführt worden sein. Dies kann vorliegend beispielsweise ein Positionieren des Patienten 2 in oder an einem C-Bogen-Röntgengerät 4 einschließen. Ebenso können Beispielsweise vor Beginn der eigentlichen medizinischen Prozedur an dem Patienten 2 bereits für das weitere Verfahren verwendbare Daten an eine Datenverarbeitungseinrichtung 5, welche das Verfahren durchführt, bereitgestellt werden. So können beispielsweise den Patienten 2 betreffende Daten, wie beispielsweise dessen Alter, Größe, Gewicht, Geschlecht und dergleichen in die Datenverarbeitungseinrichtung 5 eingegeben oder durch die Datenverarbeitungseinrichtung 5 beispielsweise aus einer elektronischen Patientenakte des Patienten 2 abgerufen werden. Ebenso kann beispielsweise eine Art und/oder ein geplanter Ablauf der durchzuführenden Prozedur an- beziehungsweise eingegeben werden. Aus diesen und/oder weiteren Daten kann durch die Datenverarbeitungseinrichtung 5 ein vorläufiges digitales Patientenmodell des Patienten 2 erzeugt werden. Ebenso kann ein derartiges vorläufiges Patientenmodell des Patienten 2 bereits vorgefertigt der Datenverarbeitungseinrichtung 5 bereitgestellt werden.

In einem Verfahrensschritt S2 werden mittels des Röntgengerätes 4 in einem medizinischen bildgebenden Verfahren Bilddaten zumindest eines Teilbereiches des Patienten 2 erzeugt beziehungsweise erfasst. Im dargestellten Beispiel wird dabei ein Brustbereich 6 des Patienten 2 abgebildet. Vorliegend beginnt damit also die eigentliche medizinische Prozedur, wobei der Arzt 1 ein medizinisches Objekt, hier einen Katheter 7 in den Patienten 2 einführt. Sobald der Katheter 7 den Brustbereich 6 erreicht, werden durch das Röntgengerät 4 also sowohl der Patient als auch zumindest ein Teilbereich des Katheters 7 abgebildet. Auch im Laufe der folgenden Verfahrensschritte, zumindest während der medizinischen Prozedur, können kontinuierlich Bilddaten des Patienten 2 und des Katheters 7 mittels des Röntgengerätes 4 aufgenommen beziehungsweise erzeugt werden.

Gleichzeitig können in einem Verfahrensschritt S3 mittels entsprechender Sensoren weitere Daten erfasst werden. Vorliegend wird dazu mittels einer Kamera 8, die bevorzugt als 3D-Kamera ausgebildet ist, der Patient 2 und eine Umgebung des Patienten 2 bildlich erfasst. Die Kamera 8 kann ebenso beispielsweise den C-Arm des Röntgengerätes 4 Erfassen. Die Kamera kann dabei beispielsweise automatisch zusammen mit dem Röntgengerät 4 oder mit der Datenverarbeitungseinrichtung 5 aktiviert werden. Von der Kamera 8 erzeugte Kameradaten, die also einen jeweiligen Teilbereich des Patienten 2, des Röntgengerätes 4 und einer Umgebung des Patienten 2 abbilden können, werden über eine entsprechende Datenverbindung an die Datenverarbeitungseinrichtung 5 übermittelt.

In einem Verfahrensschritt S4 kann die Datenverarbeitungseinrichtung 5 die mittels des Röntgengerätes 4 erzeugten Bilddaten des Patienten 2 und des Katheters 7 erfassen und unter Verwendung dieser Bilddaten ein Patientenmodell des Patienten 2 erzeugen. Das Patientenmodell kann dabei zu diesem Zeitpunkt neu erzeugt oder beispielsweise durch Weiterverarbeiten oder Aktualisieren des vorläufigen Patientenmodells erzeugt werden. Zum Erzeugen des Patientenmodells können gegebenenfalls vorab bereitgestellte Daten ebenso verwendet werden wie die von der Kamera 8 gelieferten Kameradaten. Die Kameradaten können dabei beispielsweise in einem Zwischenschritt in Geometriedaten umgerechnet werden, welche relative Lagen, Stellungen oder Positionen des Patienten 2, des Röntgengerätes 4 und/oder beispielsweise des Arztes 1 beschreiben. Auch weitere medizinische Objekte, wie beispielsweise eine Klammer oder eine Endoprothese, können erfasst und erkannt oder identifiziert werden. Derartige weitere Objekte können dann ebenfalls in dem Patientenmodell abgebildet beziehungsweise modelliert werden. Ebenso kann während der Prozedur beispielsweise zu einem oder mehreren Zeitpunkt dem Patienten 2 ein Kontrastmittel verabreicht und so ein Gefäßbaum, also ein Verlauf von Blutgefäßen des Patienten 2, sichtbar gemacht werden. Auch solche Daten, wie also etwa der Verlauf der Blutgefäße, können von der Datenverarbeitungseinrichtung 5 in das Patientenmodell eingepflegt werden. Da kontinuierlich neue Bilddaten von dem Röntgengerät 4 und/oder von der Kamera 8 geliefert werden, kann die Datenverarbeitungseinrichtung 5 das Patientenmodell ebenso kontinuierlich auf Basis der jeweils aktuellsten verfügbaren Daten aktualisieren.

Um die Verarbeitung der Bilddaten, der Kameradaten und/oder weitere bereitgestellter Daten zu vereinfachen, kann der Datenverarbeitungseinrichtung 5 manuell oder automatisch beispielsweise eine jeweilige Information über die jeweilige Datenquelle bereitgestellt werden. So kann es bevorzugt beispielsweise vorgesehen sein, dass die Datenverarbeitungseinrichtung 5 automatisch erkennen kann, von welcher beziehungsweise von welcher Art von Datenquelle jeweilige Daten beziehungsweise ein jeweiliger Datenstrom, wie etwa die Bilddaten oder die Kameradaten, stammen. Ebenso können der Datenverarbeitungseinrichtung 5 beispielsweise Daten über bereits in oder an dem Patienten 2 befindliche weitere medizinische Objekte, wie beispielsweise eine Gefäßstütze (Stent) oder dergleichen bereitgestellt werden. Derartige Daten können dann ebenfalls in das Patientenmodell einfließen.

Das Patientenmodell kann also sämtliche verfügbaren Daten bündeln.

Die Datenverarbeitungseinrichtung 5 kann die Bilddaten, beispielsweise mittels eines Bildverarbeitungsalgorithmus verarbeiten und auswerten. So können beispielsweise automatisch die Bilddaten segmentiert und/oder Objekte, insbesondere der Katheter 7, erkannt werden. Sobald der Katheter 7 erkannt worden ist, wird dessen Position in einem Verfahrensschritt S5 kontinuierlich nachverfolgt. Das Nachverfolgen wird dabei solange aufrechterhalten oder durchgeführt, wie der Katheter erfasst und erkannt wird. Der Katheter beziehungsweise dessen Position kann also auch während der nachfolgenden Verfahrensschritte nachverfolgt werden.

Da also automatisch absolute und/oder relative Positionsveränderungen beziehungsweise Bewegungen zumindest des Katheters 7 erfasst beziehungsweise erkannt und entsprechend in dem Patientenmodell abgebildet beziehungsweise repräsentiert werden, kann das Patientenmodell auch als Navigationskarte zumindest für einen Teilbereich des Patienten 2 dienen oder bezeichnet werden.

Um eine ausreichende Genauigkeit sicherzustellen, können die Position des Katheters 7 und/oder das Patientenmodell beispielsweise mit einer Frequenz von wenigstens 10 Hz aktualisiert werden.

Die jeweils aktuellen Bilddaten und/oder das jeweils aktuelle Patientenmodell kann beispielsweise mittels einer Anzeigeeinrichtung 9 in einem ersten Anzeigebereich 10 der Anzeigeeinrichtung 9 angezeigt oder dargestellt werden. Vorliegend ist hier ein Abschnitt eines Gefäßes 11 des Patienten 2 abgebildet. Das Gefäß 11 weist im vorliegenden Beispiel eine Stenose 12 auf. Weiterhin befindet sich in dem Gefäß 11 der Katheter 7 wobei eine Katheterspitze 13 des Katheters 7, also dessen vorderer Endbereich, der Stenose 12 zugewandt ist. Der Katheter 7 kann während der Prozedur von dem Arzt 1 beispielsweise auf die Stenose 12 zu bewegt werden.

In einem Verfahrensschritt S6 verarbeitet die Datenverarbeitungseinrichtung 5 zumindest die Bilddaten zumindest mittels des oder eines Bildverarbeitungsalgorithmus, um einen situativen und/oder räumlichen Kontext zu bestimmen, in dem sich der Katheter 7 jeweils zum aktuellen Zeitpunkt befindet. Zum Bestimmen dieses Kontextes können bevorzugt auch weitere der verfügbaren Daten verwendet beziehungsweise berücksichtigt werden. Dies kann beispielsweise Angaben zu der Prozedur, zu weiteren medizinischen Objekten, zum Patienten und ähnliches betreffen. Soweit noch nicht vorher zum Erzeugen des Patientenmodells geschehen, können bei dem oder zum Bestimmen des Kontextes beispielsweise wenigstens ein anatomisches Detail und/oder eine räumliche Relation bestimmt werden. So kann beispielsweise durch eine Mustererkennung und/oder einen Abgleich der Bilddaten mit einer entsprechenden Anatomiedatenbank das Gefäß 11 identifiziert und/oder die Stenose 12 als solche erkannt werden. Ebenso kann beispielsweise ein hier schematisch angedeuteter aktueller Abstand 14 von der Katheterspitze 13 zu der Stenose 12 beziehungsweise einem markierten oder automatische berechneten Mittelpunkt der Stenose 12 bestimmt beziehungsweise berechnet werden. Ebenso kann beispielsweise ein jeweils aktueller Durchmesser des Gefäßes 11 auf Höhe der Katheterspitze 13 bestimmt beziehungsweise berechnet werden.

Es kann möglich oder vorgesehen sein, dass beispielsweise der Arzt 1 oder anderes medizinisches Personal eine Markierung vorgibt. So kann beispielsweise eine bestimmte Position oder ein bestimmtes anatomisches Merkmal oder Detail markiert und/oder ein geplanter Pfad für den Katheter 7 oder dergleichen in die Bilddaten beziehungsweise deren Darstellung eingezeichnet werden. Auch solche Markierungen oder Vorgaben können von der Datenverarbeitungseinrichtung 5 zum Bestimmen des Kontextes, beispielsweise zum Bestimmen oder Berechnen eines Abstandes der Katheterspitze 13 zu einer derartigen Markierung und/oder zum Erkennen einer Abweichung eines tatsächlichen Pfades oder einer tatsächlichen Position des Katheters 7 oder der Katheterspitze 13 von dem vorgegebenen Pfad, berücksichtigt werden.

Im Verfahrensschritt S6 wird also ein faktischer und/oder sinnhafter Zusammenhang der jeweils abgebildeten aktuellen Situation, also etwa des Katheters 7 mit seiner Umgebung, die hier durch das Gefäß 11 und die Stenose 12 gegeben ist, hergestellt beziehungsweise ermittelt. Es kann also beispielsweise nicht nur der Abstand 14 als Abstand zwischen zwei geometrisch ausgezeichneten Punkten ermittelt werden, sondern es wird automatisch die Bedeutung der jeweiligen Punkte erkannt, sodass die Datenverarbeitungseinrichtung 5 also automatisch ermittelt, dass der Abstand 14 spezifisch die Entfernung zwischen der Katheterspitze und der Stenose 12 beschreibt.

Die dafür notwendige Bild- und Datenverarbeitung kann besonders vorteilhaft durch ein entsprechend trainiertes neuronales Netz durchgeführt werden. Dieses kann - beispielsweise durch Verarbeiten der Bilddaten - verschiedene Objekte, wie das Gefäß 11, die Stenose 12 und den Katheter 7 klassifizieren.

In einem Verfahrensschritt S7 werden basierend auf dem bestimmten Kontext durch die Datenverarbeitungseinrichtung 5 Sprachdaten 15, die hier schematisch angedeutet sind, erzeugt. Diese Sprachdaten 15 beschreiben wenigstens einen Teil des bestimmten Kontextes in Sprachform und werden aus dem Patientenmodell beziehungsweise aus diesem gewonnenen oder ermittelten Daten erzeugt. So können in dem Patientenmodell beispielsweise identifizierte Merkmale, Teilbereiche oder Objekte mit ihren jeweiligen sprachlich korrekten Bezeichnungen benannt beziehungsweise verknüpft werden. Die Sprachdaten 15 können somit also unter Verwendung sprachlicher Begriffe die jeweils aktuelle Situation beschreiben. Die Sprachdaten 15 können beispielsweise Sätze oder Satzfragmente sein, die abgeleitet aus dem bestimmten Kontext, gegebenenfalls unter Verwendung einer vorgegebenen Grammatik, erzeugt beziehungsweise zusammengesetzt werden. Vorliegend könnte als ein solches Sprachdatum 15 beispielsweise das Satzfragment "noch 10 mm bis zum Stenosemittelpunkt" erzeugt wird, insbesondere wenn eine Bewegung des Katheters 7 beziehungsweise der Katheterspitze 13 in Richtung der Stenose 12 als Teil des jeweils aktuellen Kontextes erkannt worden ist. Setzt der Arzt daraufhin die Bewegung des Katheters 7 in Richtung der Stenose 12 fort, so kann zu einem späteren Zeitpunkt beispielsweise als Sprachdatum 15 das Satzfragment "Mitte der Stenose erreicht" erzeugt werden. Die Sprachdaten 15 stellen also eine, insbesondere anschaulich verständliche, sprachliche Beschreibung der jeweiligen Situation, also des jeweiligen Kontextes dar. Im Verfahrensschritt S7 wird also der durch die Datenverarbeitung bestimmte Kontext beziehungsweise dessen abstrakte Repräsentation durch die Datenverarbeitungseinrichtung 5 in eine Sprachform umgewandelt.

In weiteren Anwendungsbeispielen können die Sprachdaten 15 beispielsweise eine Abweichung des Katheters 7 oder eines anderen medizinischen Objekts oder Instrumentes von einem vorbestimmten beziehungsweise geplanten oder idealen Pfad, eine Abweichung von einer entsprechenden Geschwindigkeit, eine Entfernung von einem geplanten oder vorgegebenen Appellation. Oder dergleichen mehr beschreiben beziehungsweise ausdrücken. Ebenso können die Sprachdaten 15 beispielsweise Hinweise oder Warnungen sein oder enthalten, wie beispielsweise "in 13 mm rechts abgehendes Gefäß". Derartige Angaben zu Gefäßverästelungen können beispielsweise insbesondere bei neurochirurgischen Anwendungen, wo die beteiligten Gefäße oder Gefäßverästelungen besonders fein sein können, besonders vorteilhaft und hilfreich zur Unterstützung des Arztes 1 sein.

In einem Verfahrensschritt S8 werden die Sprachdaten 15 dann an das medizinische Personal, hier also beispielsweise an den Arzt 1, ausgegeben. Dazu können die Sprachdaten 15 beispielsweise in einem zweiten Anzeigebereich 16 der Anzeigeeinrichtung 9 als Text angezeigt werden. Bevorzugt können die Sprachdaten 15 jedoch mittels einer Lautsprechereinrichtung 17 in akustischer Form, also als akustische Sprachausgabe, aus- oder wiedergegeben werden. Hierdurch kann sich der Arzt 1 beispielsweise auf die jeweilige klinische Fragestellung konzentrieren, wird weniger abgelenkt und verfügt vorteilhaft über eine zusätzliche Absicherung. Ebenso kann sich weiteres medizinisches Personal durch die ausgegebenen Sprachdaten 15 vorausschauend auf bevorstehende Abläufe, Handreichungen und dergleichen selbstständig vorbereiten, da quasi jeweilige Gedanken, die der Arzt 1 während der Prozedur üblicherweise hat, auch für das übrige medizinische Personal hörbar gemacht werden. Insbesondere die akustische Sprachausgabe mittels der Lautsprechereinrichtung 17 ermöglicht es dabei vorteilhaft, sämtliches medizinisches Personal besonders einfach und zeitnah automatisch zu informieren.

Die Sprachdaten 15 können dabei nicht nur in dem jeweiligen Operationssaal, sondern ebenso an anderer Stelle ausgegeben werden. Die Sprachdaten 15 können beispielsweise zu Lehrzwecken oder im Rahmen einer Konsultation sich entfernt aufhaltender Fachkräfte, zum Beispiel in einem Beobachtungsraum, in einem Hörsaal, in einem Fahrzeug, auf einem mobilen Endgerät oder dergleichen ausgegeben werden. Die Sprachdaten 15 können dabei einfacher und schneller an einen jeweiligen Ausgabeort übermittelt werden, als beispielsweise die Bilddaten. Zudem können je nach Möglichkeiten und Darstellungsqualität einer jeweiligen entfernten Anzeigeeinrichtung oder eines jeweiligen entfernten Anzeigegerätes die Bilddaten nur schwierig zuverlässig erkennbar oder interpretierbar sein, während die Sprachdaten 15 deutlich einfacher und zuverlässiger korrekt ausgebbar und verstehbar sind.

Das Erzeugen und Ausgeben der Sprachdaten 15 kann beispielsweise auch dann besonders vorteilhaft sein, wenn medizinisches Personal unterschiedlicher Qualifikationen anwesend, insbesondere an der jeweiligen medizinischen Prozedur beteiligt ist. So können beispielsweise ein Radiologe und ein Chirurg während der medizinischen Prozedur kooperieren, wobei beispielsweise nicht beide über dieselbe Erfahrung oder dasselbe Wissen im Zusammenhang mit sämtlichen Schritten der Prozedur und/oder dem Interpretieren einer rein bildlichen Darstellung der von dem Röntgengerät 4 gelieferten Bilddaten verfügen. Durch die Ausgabe der Sprachdaten 15 kann dann sichergestellt werden, dass die jeweilige Situation besonders klar und einfach verständlich vermittelt wird.

In einem Verfahrensschritt S9 können beispielsweise bestimmte oder erkannte Abweichungen des Patienten 2 von einem Normalverhalten oder Normalzustand in einer Abweichungsdatenbank gespeichert werden. Ebenso kann auf diese Abweichungsdatenbank zum Bestimmen des Kontextes und/oder zum Erzeugen oder Aktualisieren des Patientenmodells zugegriffen werden.

Das System zum Unterstützen des medizinischen Personals beziehungsweise des Arztes 1 in der beschriebenen Art und Weise umfasst zumindest die Datenverarbeitungseinrichtung 5. Die Datenverarbeitungseinrichtung 5 kann dabei jeweilige Schnittstellen zum Empfangen und/oder zum Übermitteln von Daten, also zum einseitigen oder beidseitigen Datenaustausch mit dem Röntgengerät 4, der Kamera 8, der Anzeigeeinrichtung 9 und/oder der Lautsprechereinrichtung 17 aufweisen. Ebenso können die Kamera 8, die Anzeigeeinrichtung 9 und/oder die Lautsprechereinrichtung 17 Teil des Systems sein.

Das System kann als eigenständiges separates System oder Gerät ausgebildet sein. Ebenso kann es jedoch möglich sein, das System in ein größeres System oder ein anderes Gerät zu integrieren. So kann das System, insbesondere die Datenverarbeitungseinrichtung 5, beispielsweise in eine Datenverarbeitungsvorrichtung integriert oder mit einer Datenverarbeitungsvorrichtung kombiniert sein, welche von dem Röntgengerät 4 bereitgestellte Rohdaten zu den Bilddaten rekonstruiert.

Die beschriebenen Verfahrensschritte sind nicht streng als Abfolge von zeitlich aufeinanderfolgenden Vorgängen zu verstehen. Vielmehr können im Zusammenhang mit unterschiedlichen Verfahrensschritten beschriebene Vorgänge oder Maßnahmen gegebenenfalls gleichzeitig ausgeführt werden und/oder sich in ihrer Dauer oder Ausführungszeit überschneiden, also zeitlich überlappen.

Das beschriebene Verarbeiten von unterschiedlichen Daten und Informationen zu sprachlichen Informationsaussagen kann insbesondere, aber nicht ausschließlich, für interventionell unterstützte medizinische Prozeduren eine vorteilhafte Maßnahme beziehungsweise Lösung zur Unterstützung von medizinischem Personal und zur Verbesserung eines Ablaufs der jeweiligen Prozedur sein.

## Patentansprüche

1. Verfahren (3) zur Unterstützung von medizinischem Personal (1) bei einer Prozedur an einem Patienten (2), mit den Verfahrensschritten
- kontinuierliches Erfassen (S3) von mittels eines medizinischen bildgebenden Verfahrens erzeugten Bilddaten des Patienten (2) und eines medizinischen Objekts (7, 13),
- Erzeugen und kontinuierliches Aktualisieren (S4) eines digitalen Patientenmodells unter Verwendung der Bilddaten,
- kontinuierliches Nachverfolgen (S5) einer Position des medizinischen Objekts (7, 13) aus den Bilddaten,
- Bestimmen (S6) eines situativen und räumlichen Kontextes, in dem sich das medizinische Objekt (7, 13) befindet, durch Auswertung des Patientenmodells und automatisches Verarbeiten der Bilddaten mittels eines Bildverarbeitungsalgorithmus, wobei der situative Kontext durch die Art der jeweils aktuell durchgeführten Prozedur, die Art des medizinischen Objekts oder einen Zustand des Patienten gegeben sein kann,
- basierend auf dem bestimmten Kontext aus dem Patientenmodell automatisches Erzeugen (S7) von Sprachdaten (15), die wenigstens einen Teil des bestimmten Kontextes in Sprachform beschreiben, und
- Ausgeben (S8) der Sprachdaten (15) an das medizinische Personal (1) mittels einer akustischen Sprachausgabe und/oder als Text auf einer Anzeigefläche (9, 16),
**dadurch gekennzeichnet, dass**
- als das medizinische Objekt ein Katheter verwendet wird,
und
- unterschiedliche Sprachdaten erzeugt und in Abhängigkeit von ihrem Inhalt in unterschiedliche Kategorien betreffend ein Anatomiemerkmal oder das medizinische Objekt eingeordnet werden, und
- nur Sprachdaten aus ausgewählten der Kategorien ausgegeben werden.

2. Verfahren (3) nach Anspruch 1, **dadurch gekennzeichnet, dass** als der Kontext ein abgebildeter Teilbereich (6) des Patienten (2) und/oder ein anatomisches Merkmal (11, 12) des Patienten (2) und/oder eine räumlich-geometrische Relation zwischen dem medizinischen Objekt (7, 13) und wenigstens einem Umgebungsdetail (11, 12), insbesondere ein Abstand zu einem vorbestimmten und/oder automatisch erkannten anatomischen Merkmal (11, 12) des Patienten (2), in einer Umgebung des medizinischen Objekts (7, 13) bestimmt wird.

3. Verfahren (3) nach einem der vorhergehenden Ansprüche, **dadurch gekennzeichnet, dass** der Kontext bestimmt wird durch einen automatischen Abgleich der Bilddaten und/oder des Patientenmodells mit einer bereitgestellten Anatomiedatenbank.

4. Verfahren (3) nach einem der vorhergehenden Ansprüche, **dadurch gekennzeichnet, dass** zum Erzeugen des Patientenmodells und/oder zum Bestimmen des Kontextes eine Lage des Patienten (2) und/oder dessen Umgebung, insbesondere eine relative Position eines Patientenlagers und/oder eines die Bilddaten generierenden Geräts (4), beschreibende Geometriedaten verwendet werden.

5. Verfahren (3) nach einem der vorhergehenden Ansprüche, **dadurch gekennzeichnet, dass** mittels einer 3D-Kamera (8) der Patient (2) und/oder eine Umgebung des Patienten (2) räumlich erfasst wird und entsprechende von der 3D-Kamera (8) bereitgestellte Kameradaten zum Erzeugen des Patientenmodells und/oder zum Bestimmen des Kontexts verarbeitet werden.

6. Verfahren (3) nach einem der vorhergehenden Ansprüche, **dadurch gekennzeichnet, dass**
- das medizinische Objekt (7, 13) mittels eines automatischen Objekterkennungsalgorithmus und/oder durch einen automatischen Abgleich der Bilddaten mit einer bereitgestellten Objektdatenbank identifiziert wird, und
- dem identifizierten Objekt (7, 13) zugeordnete Objektdaten aus der Objektdatenbank abgerufen und zum Erzeugen des Patientenmodells und/oder zum Bestimmen des Kontextes verarbeitet werden.

7. Verfahren (3) nach einem der vorhergehenden Ansprüche, **dadurch gekennzeichnet, dass**
- während der Katheter (7, 13) durch ein Gefäß (11) des Patienten (2) geführt wird, mittels eines Bildverarbeitungsalgorithmus automatisch ein Durchmesser des Gefäßes (11) an einer jeweils aktuellen Position des Katheters (7, 13), insbesondere einer Spitze (13) des Katheters (7), bestimmt wird.

8. Verfahren (3) nach einem der vorhergehenden Ansprüche, **dadurch gekennzeichnet, dass**
- bei dem kontinuierlichen Erfassen der Bilddaten zwischen zwei, insbesondere zu Dokumentationszwecken, aufgenommenen Dokumentationsbildern des Patienten (2) eine Vielzahl von Durchleuchtungsbildern mit reduzierter Strahlungsintensität aufgenommen werden, und
- diese Durchleuchtungsbilder zum Aktualisieren des Patientenmodells verarbeitet werden.

9. Verfahren (3) nach einem der vorhergehenden Ansprüche, **dadurch gekennzeichnet, dass**
- mittels einer Methode des maschinellen Lernens, insbesondere mittels eines neuronalen Netzes, die Bilddaten und/oder die Patientenmodelle einer Vielzahl von verschiedenen Patienten (2) verarbeitet und dadurch ein Normalzustand und/oder ein Normalverhalten wenigstens eines Teils (6, 11, 12) des Patienten (2 gelernt wird,
- basierend darauf automatisch eine Abweichung zwischen den jeweils aktuellen Bilddaten und/oder dem jeweils aktuellen Patientenmodell einerseits und dem Normalzustand und/oder dem Normalverhalten andererseits erkannt, und
- die Abweichung beschreibende Sprachdaten (15) erzeugt und ausgegeben werden und/oder
- die jeweils aktuellen Bilddaten und/oder das jeweils aktuelle Patientenmodell in einer Abweichungsdatenbank gespeichert werden (S9).

10. System (5) zur Unterstützung von medizinischem Personal (1) bei einer Prozedur an einem Patienten (2), aufweisend
- eine Erfassungseinrichtung (4, 5), zum kontinuierlichen Erfassen von mittels eines medizinischen bildgebenden Verfahrens erzeugten Bilddaten des Patienten (2) und eines medizinischen Objekts (7, 13),
- eine Datenverarbeitungseinrichtung (5), zum
- Erzeugen und kontinuierlichen Aktualisieren eines digitalen Patientenmodells auf Basis der jeweils aktuellen Bilddaten,
- Nachverfolgen einer Position des medizinischen Objekts (7, 13) aus den Bilddaten, und
- automatischen Bestimmen eines situativen und räumlichen Kontexts, in denen sich das medizinische Objekt (7, 13) befindet, durch Auswertung des Patientenmodells und Verarbeiten der Bilddaten mittels eines Bildverarbeitungsalgorithmus, wobei der situative Kontext durch die Art der jeweils aktuell durchgeführten Prozedur, die Art des medizinischen Objekts oder einen Zustand des Patienten gegeben sein kann,
- eine Spracherzeugungseinrichtung (5), zum Erzeugen von Sprachdaten (15) basierend auf dem bestimmten Kontext aus dem Patientenmodell, wobei die Sprachdaten (15) wenigstens einen Teil des bestimmten Kontextes in Sprachform beschreiben, und
- eine Ausgabeeinrichtung (9, 17), zum Ausgeben der Sprachdaten (15) an das medizinische Personal (1) mittels einer akustischen Sprachausgabe und/oder als Text auf einer Anzeigefläche der Ausgabeeinrichtung (9)
**dadurch gekennzeichnet, dass**
- als das medizinische Objekt ein Katheter verwendet wird,
und
- unterschiedliche Sprachdaten erzeugt und in Abhängigkeit von ihrem Inhalt in unterschiedliche Kategorien betreffend ein Anatomiemerkmal oder das medizinische Objekt eingeordnet werden, und
- nur Sprachdaten aus ausgewählten der Kategorien ausgegeben werden.

## Claims

1. Method (3) for supporting medical personnel (1) in a procedure on a patient (2), having the steps
- continuously capturing (S3) image data of the patient (2) and of a medical object (7, 13) generated by means of a medical imaging method,
- generating and continuously updating (S4) a digital patient model, using the image data,
- continuously tracking (S5) a position of the medical object (7, 13) from the image data,
- determining (S6) a situational and spatial context in which the medical object (7, 13) is situated by evaluation of the patient model and automatic processing of the image data by means of an image processing algorithm, wherein the situational context can be specified by the type of the respective currently performed procedure, the type of medical object or a status of the patient,
- based on the determined context from the patient model, automatically generating (S7) speech data (15) which describes at least part of the determined context in speech form, and
- outputting (S8) the speech data (15) to the medical personnel (1) by means of an acoustic speech output and/or as text on a display surface (9, 16),
**characterised in that**
- a catheter is used as the medical object, and
- different speech data is generated and classified into different categories dependent upon its content relating to an anatomic feature or the medical object, and
- only speech data from selected categories is output.

2. Method (3) according to claim 1, **characterised in that** as the context, a represented subregion (6) of the patient (2) and/or an anatomical feature (11, 12) of the patient (2) and/or a spatial-geometric relation between the medical object (7, 13) and at least one environmental detail (11, 12), in particular a spacing from a pre-determined and/or automatically recognized anatomical feature (11, 12) of the patient (2) in an environment of the medical object (7, 13), is determined.

3. Method (3) according to one of the preceding claims, **characterised in that** the context is determined by an automatic comparison of the image data and/or of the patient model with an anatomical database provided.

4. Method (3) according to one of the preceding claims, **characterised in that**, in order to generate the patient model and/or to determine the context, geometrical data describing a location of the patient (2) and/or his environment, in particular a relative position of a patient couch and/or of a device (4) generating the image data is used.

5. Method (3) according to one of the preceding claims, **characterised in that** the patient (2) and/or an environment of the patient (2) are spatially captured by means of a 3D camera (8) and corresponding camera data provided by the 3D camera (8) is processed to generate the patient model and/or to determine the context.

6. Method (3) according to one of the preceding claims, **characterised in that**
- the medical object (7, 13) is identified by means of an automatic object recognition algorithm and/or by an automatic comparison of the image data with an object database provided, and
- object data assigned to the identified object (7, 13) is recalled from the object database and processed to generate the patient model and/or to determine the context.

7. Method (3) according to one of the preceding claims, **characterised in that**
- while the catheter (7, 13) is passed through a vessel (11) of the patient (2), a diameter of the vessel (11) at a respective current position of the catheter (7, 13), in particular a tip (13) of the catheter (7), is automatically determined by means of an image processing algorithm.

8. Method (3) according to one of the preceding claims, **characterised in that**
- on continuous capture of the image data between two documentation images of the patient (2) recorded, in particular for documentation purposes, a plurality of fluoroscopic images with reduced radiation intensity is recorded, and
- these fluoroscopic images are processed for updating the patient model.

9. Method (3) according to one of the preceding claims, **characterised in that**
- by means of a method of machine learning, in particular by means of a neural network, the image data and/or the patient models of a plurality of different patients (2) are processed and thereby a normal state and/or a normal behaviour of at least a part (6, 11, 12) of the patient (2) is learned,
- on the basis thereof, a deviation between, firstly, the respective current image data and/or the respective current patient model and, secondly, the normal state and/or the normal behaviour is automatically recognized, and
- speech data (15) describing the deviation is generated and output, and/or
- the respective current image data and/or the respective current patient model is stored in a deviation database (S9) .

10. System (5) for supporting medical personnel (1) in a procedure on a patient (2), comprising
- a capture device (4, 5) for continuously capturing image data of the patient (2) and of a medical object (7, 13) generated by at least one medical imaging method,
- a data processing device (5) for
- generating and continuously updating a digital patient model on the basis of the respective current image data,
- tracking of a position of the medical object (7, 13), and
- automatically determining a situational and spatial context in which the medical object (7, 13) is situated, by evaluation of the patient model and processing of the image data by means of an image processing algorithm, wherein the situational context can be specified by the type of the respective currently performed procedure, the type of medical object or a status of the patient
- a speech synthesis device (5) for generating speech data (15) on the basis of the determined context from the patient model, wherein the speech data (15) describes at least part of the particular context in speech form, and
- an output device (9, 17) for outputting the speech data (15) to the medical personnel (1) by means of an acoustic speech output and/or as text on a display surface of the output device (9),
**characterised in that**
- a catheter is used as the medical object, and
- different speech data is generated and classified into different categories dependent upon its content relating to an anatomic feature or the medical object, and
- only speech data from selected categories is output.

## Revendications

1. Procédé (3) pour porter assistance au personnel (1) médical, lors d'une procédure sur un patient (2), comprenant les stades de procédé
- saisie (S3) en continu de données d'image produites au moyen d'un procédé d'imagerie médicale du patient (2) et d'un objet (7, 13) médical,
- production et mise à jour (S4) en continu d'un modèle numérique du patient en utilisant les données d'image,
- suivi (S5) en continu d'une position de l'objet (7, 13) médical à partir des données d'image,
- détermination (S6) d'un contexte situationnel et spatial, dans lequel se trouve l'objet (7, 13) médical, en exploitant le modèle du patient et en traitant automatiquement les données d'image au moyen d'un algorithme de traitement d'image, dans lequel le contexte situationnel peut être donné par le type de procédure effectuée en cours respectivement, le type de l'objet médical ou un état du patient,
- sur la base du contexte déterminé, production (S7) automatique, à partir du modèle du patient, de données (15) vocales, qui décrivent sous forme vocale au moins une partie du contexte déterminé, et
- donnée (S8) des données (15) vocales au personnel (1) médical au moyen d'une émission vocale acoustique et/ou sous la forme d'un texte sur une surface (9, 16) d'affichage,
**caractérisé en ce que**
- l'on utilise un cathéter comme objet médical, et
- l'on produit des données vocales différentes et, en fonction de leur contenu, on les range dans des catégories différentes concernant une caractéristique anatomique ou l'objet médical, et
- l'on ne donne des données vocales que de catégories sélectionnées parmi les catégories.

2. Procédé (3) suivant la revendication 1, **caractérisé en ce que** l'on détermine, dans un environnement de l'objet (7, 13) médical, comme le contexte, une région (6) partielle représentée du patient (2) et/ou une caractéristique (11, 12) anatomique du patient (2) et/ou une relation géométrique dans l'espace entre l'objet (7, 13) médical et au moins un détail (11, 12) ambiant, en particulier une distance à une caractéristique (11, 12) anatomique du patient (2) déterminée à l'avance et/ou détectée automatiquement.

3. Procédé (3) suivant l'une des revendications précédentes, **caractérisé en ce que** l'on détermine le contexte par une correspondance automatique des données d'image et/ou du modèle du patient avec une base de données anatomiques mise à disposition.

4. Procédé (3) suivant l'une des revendications précédentes, **caractérisé en ce que**, pour la production du modèle du patient et/ou pour la détermination du contexte, on utilise une position du patient (2) et/ou de son environnement, en particulier des données géométriques décrivant une position relative d'une couchette du patient et/ou d'un appareil (4) produisant les données d'image.

5. Procédé (3) suivant l'une des revendications précédentes, **caractérisé en ce que**, au moyen d'une caméra (8) en 3D, on prend dans l'espace le patient (2) et/ou un environnement du patient (2) et on les traite, conformément aux données de caméra mises à disposition par la caméra (8) en 3D, pour la production du modèle du patient et/ou pour la détermination du contexte.

6. Procédé (3) suivant l'une des revendications précédentes, **caractérisé en ce que**
- l'on identifie l'objet (7, 13) médical au moyen d'un algorithme de détection automatique d'objet et/ou par une correspondance automatique des données d'image avec une base de données d'objet mise à disposition, et
- l'on appelle, de la base de données d'objet, des données d'objet associées à l'objet (7, 13) identifié et on les traite pour la production du modèle du patient et/ou pour la détermination du contexte.

7. Procédé (3) suivant l'une des revendications précédentes, **caractérisé en ce que**
- pendant que l'on fait passer le cathéter (7, 13) dans un vaisseau (11) du patient (2), on détermine, au moyen d'un algorithme de traitement d'image, automatiquement un diamètre du vaisseau (11) en une position en cours respective du cathéter (7, 13), en particulier d'une pointe (13) du cathéter (7) .

8. Procédé (3) suivant l'une des revendications précédentes, **caractérisé en ce que**
- lors de la saisie en continu des données d'image, on enregistre, entre deux images de documentation du patient (2) enregistrées, en particulier à des fins de documentation, une pluralité d'images de radioscopie avec une intensité du rayonnement réduite, et
- l'on traite ces images de radioscopie pour la mise à jour du modèle du patient.

9. Procédé (3) suivant l'une des revendications précédentes, **caractérisé en ce que**
- au moyen d'une méthode de l'apprentissage automatique, en particulier au moyen d'un réseau neuronal, on traite les données d'image et/ou les modèles de patient d'une pluralité de patients (2) différents et on apprend ainsi un état normal et/ou un comportement normal d'au moins une partie (6, 11, 12) du patient (2),
- sur cette base, on détecte automatiquement un écart entre les données d'image en cours respectives et/ou le modèle du patient en cours respectif d'une part et l'état normal et/ou le comportement normal d'autre part, et
- l'on produit des données (15) vocales décrivant l'écart et on les donne et/ou
- l'on met en mémoire (S9) les données d'image en cours respectives et/ou le modèle de patient en cours respectif dans une base de données d'écart.

10. Système (5) pour prêter assistance au personnel (1) médical, lors d'une procédure sur un patient (2), comportant
- un dispositif (4, 5) de saisie, pour la saisie en continu de données d'image du patient (2) produites au moyen d'un procédé d'imagerie médicale et d'un objet (7, 13) médical,
- un dispositif (5) de traitement de données, pour
- produire et mettre à jour en continu d'un modèle numérique du patient sur la base des données d'image en cours,
- suivre une position de l'objet (7, 13) médical à partir des données d'image, et
- déterminer un contexte situationnel et spatial, dans lequel se trouve l'objet (7, 13) médical, en exploitant le modèle du patient et en traitant automatiquement les données d'image au moyen d'un algorithme de traitement d'image, dans lequel le contexte situationnel peut être donné par le type de procédure effectuée en cours respectivement, le type de l'objet médical ou un état du patient,
- un dispositif (5) de production vocale, pour la production de données (15) vocales sur la base du contexte déterminé à partir du modèle du patient, dans lequel les données (15) vocales décrivent sous forme vocale au moins une partie du contexte déterminé, et
- un dispositif (9, 17) de donnée, pour l'émission des données (15) vocales au personnel (1) médical, au moyen d'une émission vocale acoustique et/ou sous la forme d'un texte sur une surface d'affichage du dispositif (9) d'émission,
**caractérisé en ce que**
- l'on utilise un cathéter comme objet médical, et
- l'on produit des données vocales différentes et, en fonction de leur contenu, on les range dans des catégories différentes concernant une caractéristique anatomique ou l'objet médical, et
- l'on ne donne des données vocales que de catégories sélectionnées parmi les catégories.
